(19) Europäisches Patentamt
European Patent Office
Office européen des brevets

(11) EP 3 735 991 A1

(12) DEMANDE DE BREVET EUROPEEN

(43) Date de publication:
**11.11.2020 Bulletin 2020/46**

(51) Int Cl.:
***A61K 47/68*** *(2017.01)* ***A61P 35/00*** *(2006.01)*

(21) Numéro de dépôt: **19305578.7**

(22) Date de dépôt: **06.05.2019**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(71) Demandeur: **PIERRE FABRE MEDICAMENT**
**92100 Boulogne-Billancourt (FR)**

(72) Inventeurs:
- **JOUHANNEAUD, Alexandra**
  **34000 Montpellier (FR)**
- **GOETSCH, Liliane**
  **74130 Ayze (FR)**

(74) Mandataire: **Regimbeau**
**20, rue de Chazelles**
**75847 Paris Cedex 17 (FR)**

(54) **ADC POUR UN TRAITEMENT CONCOMITANT OU POSTÉRIEUR AU DOCÉTAXEL**

(57) La présente invention concerne un ADC de formule (I) suivante

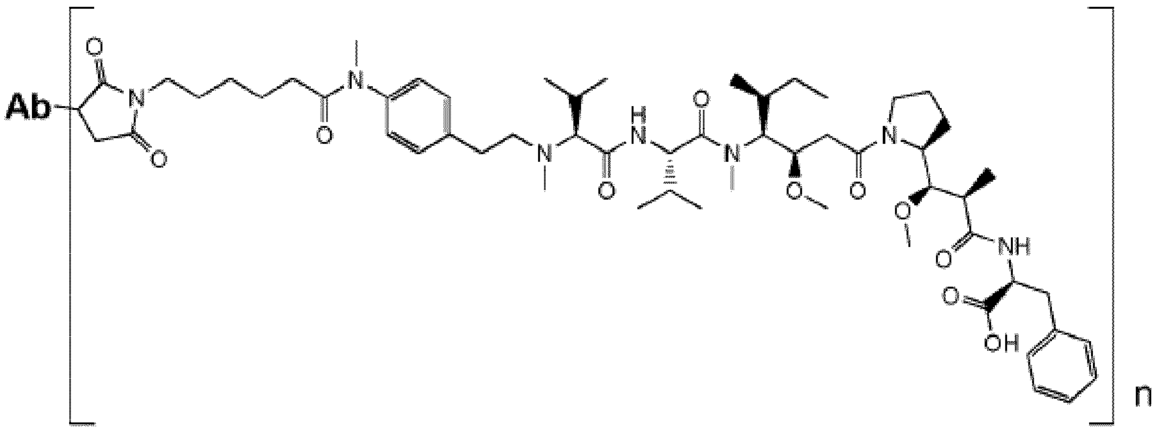

(I)

dans laquelle Ab est un anticorps anti-IGF1R caractérisé en ce qu'il comprend des CDR de la chaine légère de séquences SEQ ID Nos. 9, 5 et 11 respectivement et des CDR de chaine lourde de séquence SEQ ID Nos 7, 2 et 3 respectivement, et n est compris entre 1 et 12,
pour son utilisation dans le traitement de maladies prolifératives, telle que le cancer, caractérisée en ce que l'ADC est administré de façon concomitante ou postérieurement à du docétaxel.

EP 3 735 991 A1

SEQUENCE LISTING

<110> PIERRE FABRE MEDICAMENT

<120> ADC POUR UN TRAITEMENT CONCOMITANT OU POSTERIEUR AU DOCETAXEL

<130> B75300D39521

<160> 78

<170> PatentIn version 3.5

<210> 1
<211> 8
<212> PRT
<213> artificial

<220>
<223> Consensus CDR -H1

<220>
<221> MISC_FEATURE
<222> (3)..(3)
<223> Thr may be replaced by Ser

<220>
<221> MISC_FEATURE
<222> (8)..(8)
<223> Tyr may be replaced by Phe

<400> 1

Gly Tyr Thr Phe Thr Ser Tyr Tyr
1               5

<210> 2
<211> 8
<212> PRT
<213> artificial

<220>
<223> Consensus CDR-H2

<400> 2

Ile Trp Pro Gly Asp Gly Ser Thr
1               5

<210> 3
<211> 13
<212> PRT
<213> artificial

<220>
<223> Consensus CDR-H3

<400> 3

Ala Ser Pro Met Ile Thr Pro Asn Tyr Ala Met Asp Tyr
1               5                   10

<210> 4
<211> 6
<212> PRT
<213> artificial

<220>
<223> Consensus CDR-L1

<220>
<221> MISC_FEATURE
<222> (4)..(4)
<223> Ser may be replaced by Asn

<400> 4

Gln Asp Ile Ser Lys Tyr
1 5

<210> 5
<211> 3
<212> PRT
<213> artificial

<220>
<223> Consensus CDR-L2

<400> 5

Tyr Thr Ser
1

<210> 6
<211> 9
<212> PRT
<213> artificial

<220>
<223> Consensus CDR-L3

<220>
<221> MISC_FEATURE
<222> (5)..(5)
<223> Thr may be replaced by Ala

<400> 6

Gln Gln Gly Ser Thr Leu Pro Tyr Thr
1 5

<210> 7
<211> 8
<212> PRT
<213> artificial

<220>
<223> CDR-H1

<400> 7

Gly Tyr Thr Phe Thr Ser Tyr Tyr
1 5

<210> 8
<211> 8
<212> PRT
<213> artificial

<220>
<223> CDR-H1

<400> 8

Gly Tyr Ser Phe Thr Ser Tyr Phe
1 5

<210> 9
<211> 6
<212> PRT
<213> artificial

<220>
<223> CDR-L1

<400> 9

Gln Asp Ile Ser Lys Tyr
1 5

<210> 10
<211> 6
<212> PRT
<213> artificial

<220>
<223> CDR-L1

<400> 10

Gln Asp Ile Asn Lys Tyr
1 5

<210> 11
<211> 9
<212> PRT
<213> artificial

<220>
<223> CDR-L3

<400> 11

Gln Gln Gly Ser Thr Leu Pro Tyr Thr
1 5

<210> 12
<211> 9
<212> PRT
<213> artificial

<220>
<223> CDR-L3

<400> 12

Gln Gln Gly Ser Ala Leu Pro Tyr Thr
1 5

<210> 13
<211> 120
<212> PRT
<213> artificial

<220>
<223> c208F2, heavy chain, VH

<400> 13

Gln Val Gln Leu Gln Gln Ser Gly Pro Glu Leu Val Lys Pro Gly Ala
1 5 10 15

Ser Val Lys Met Ser Cys Lys Ala Ser Gly Tyr Thr Phe Thr Ser Tyr
20 25 30

Tyr Ile Tyr Trp Val Lys Gln Arg Pro Gly Gln Gly Leu Glu Trp Leu
35 40 45

Gly Trp Ile Trp Pro Gly Asp Gly Ser Thr Lys Tyr Asn Glu Lys Phe
50 55 60

Lys Asp Lys Thr Thr Leu Thr Ala Asp Lys Ser Ser Asn Thr Ala Tyr
65 70 75 80

Met Phe Leu Ser Ser Leu Thr Ser Glu Asp Ser Ala Val Tyr Phe Cys
85 90 95

Ala Ser Pro Met Ile Thr Pro Asn Tyr Ala Met Asp Tyr Trp Gly Gln
100 105 110

Gly Ala Ser Val Thr Val Ser Ser
115 120

<210> 14
<211> 120
<212> PRT
<213> artificial

<220>
<223> c212A11, heavy chain, VH

<400> 14

Gln Val Gln Leu Gln Gln Ser Gly Pro Glu Leu Val Lys Pro Gly Ala
1 5 10 15

Ser Val Lys Met Ser Cys Lys Ala Ser Gly Tyr Thr Phe Thr Ser Tyr
20 25 30

Tyr Ile His Trp Val Lys Gln Arg Pro Gly Gln Gly Leu Glu Trp Ile
35 40 45

Gly Trp Ile Trp Pro Gly Asp Gly Ser Thr Lys Tyr Asn Glu Lys Phe
50 55 60

Lys Gly Lys Thr Thr Leu Thr Ala Asp Lys Ser Ser Ser Thr Ala Tyr
65 70 75 80

Met Phe Leu Ser Ser Leu Thr Ser Glu Asp Ser Ala Val Tyr Phe Cys
85 90 95

Ala Ser Pro Met Ile Thr Pro Asn Tyr Ala Met Asp Tyr Trp Gly Gln
100 105 110

Gly Ala Ser Val Thr Val Ser Ser
115 120

<210> 15
<211> 120
<212> PRT
<213> artificial

<220>
<223> c214F8, heavy chain, VH

<400> 15

Gln Val Gln Leu Gln Gln Ser Gly Ser Glu Leu Val Lys Pro Gly Ala
1 5 10 15

Ser Val Lys Leu Ser Cys Lys Ala Ser Gly Tyr Thr Phe Thr Ser Tyr
20 25 30

Tyr Ile His Trp Val Lys Gln Arg Pro Gly Gln Gly Leu Glu Trp Ile
35 40 45

Gly Trp Ile Trp Pro Gly Asp Gly Ser Thr Lys Tyr Asn Glu Arg Phe
50 55 60

Lys Gly Lys Thr Thr Leu Thr Ala Asp Lys Ser Ser Ser Thr Ala Tyr
65 70 75 80

```
Met Phe Leu Ser Ser Leu Thr Ser Glu Asp Ser Ala Val Tyr Phe Cys
                85                  90                  95

Ala Ser Pro Met Ile Thr Pro Asn Tyr Ala Met Asp Tyr Trp Gly Gln
            100                 105                 110

Gly Ala Ser Val Thr Val Ser Ser
        115                 120
```

<210> 16
<211> 120
<212> PRT
<213> artificial

<220>
<223> c219D6, heavy chain, VH

<400> 16

```
Gln Val Gln Leu Gln Gln Ser Gly Pro Glu Leu Val Lys Pro Gly Asp
1               5                   10                  15

Ser Val Lys Met Ser Cys Lys Ala Ser Gly Tyr Ser Phe Thr Ser Tyr
            20                  25                  30

Phe Ile His Trp Val Lys Gln Arg Pro Gly Gln Gly Leu Glu Trp Ile
        35                  40                  45

Gly Trp Ile Trp Pro Gly Asp Gly Ser Thr Lys Tyr Asn Glu Lys Phe
    50                  55                  60

Lys Gly Lys Thr Thr Leu Thr Thr Asp Lys Ser Ser Ser Thr Ala Tyr
65                  70                  75                  80

Met Phe Leu Asn Ser Leu Thr Ser Glu Asp Ser Ala Val Tyr Phe Cys
                85                  90                  95

Ala Ser Pro Met Ile Thr Pro Asn Tyr Ala Met Asp Tyr Trp Gly Gln
            100                 105                 110

Gly Ala Ser Val Thr Val Ser Ser
        115                 120
```

<210> 17
<211> 120
<212> PRT
<213> artificial

<220>
<223> c213B10, heavy chain, VH

<400> 17

Gln Val Gln Leu Gln Gln Ser Gly Ser Glu Leu Val Lys Pro Gly Ala
1 5 10 15

Ser Val Lys Met Ser Cys Lys Ala Ser Gly Tyr Thr Phe Thr Ser Tyr
20 25 30

Tyr Ile His Trp Val Lys Gln Arg Pro Gly Gln Gly Leu Glu Trp Ile
35 40 45

Gly Trp Ile Trp Pro Gly Asp Gly Ser Thr Lys Tyr Asn Glu Arg Phe
50 55 60

Lys Gly Lys Thr Thr Leu Thr Ala Asp Lys Ser Ser Ser Thr Ala Tyr
65 70 75 80

Met Phe Leu Ser Ser Leu Thr Ser Glu Asp Ser Ala Val Tyr Phe Cys
85 90 95

Ala Ser Pro Met Ile Thr Pro Asn Tyr Ala Met Asp Tyr Trp Gly Gln
100 105 110

Gly Ala Ser Val Thr Val Ser Ser
115 120

<210> 18
<211> 107
<212> PRT
<213> artificial

<220>
<223> c208F2, light chain, VL

<400> 18

Asp Ile Gln Met Thr Gln Thr Thr Ser Ser Leu Ser Ala Ser Leu Gly
1 5 10 15

Asp Arg Val Thr Ile Ser Cys Arg Ala Ser Gln Asp Ile Ser Lys Tyr
20 25 30

Leu Asn Trp Tyr Gln Gln Gln Pro Asp Gly Thr Ile Lys Leu Leu Ile
35 40 45

Tyr Tyr Thr Ser Arg Leu His Ser Gly Val Pro Ser Arg Phe Ser Gly
50 55 60

Arg Gly Ser Gly Thr Asp Tyr Ser Leu Thr Ile Ser Asn Val Glu Gln
65 70 75 80

Glu Asp Ile Ala Thr Tyr Phe Cys Gln Gln Gly Ser Thr Leu Pro Tyr
85 90 95

Thr Phe Gly Gly Gly Thr Lys Leu Glu Ile Lys
100 105

<210> 19
<211> 107
<212> PRT
<213> artificial

<220>
<223> c212A11, light chain, VL

<400> 19

Asp Ile Gln Met Thr Gln Thr Thr Ser Ser Leu Ser Ala Ser Leu Gly
1 5 10 15

Asp Arg Val Thr Ile Ser Cys Arg Ala Ser Gln Asp Ile Asn Lys Tyr
20 25 30

Leu Asn Trp Tyr Gln Gln Gln Pro Asp Gly Thr Val Lys Leu Leu Ile
35 40 45

Tyr Tyr Thr Ser Arg Leu His Ser Gly Val Pro Ser Arg Phe Ser Gly
50 55 60

Arg Gly Ser Gly Thr Asp Tyr Ser Leu Thr Ile Ser Asn Leu Glu Gln
65 70 75 80

Glu Asp Ile Ala Thr Tyr Phe Cys Gln Gln Gly Ser Thr Leu Pro Tyr
85 90 95

Thr Phe Gly Gly Gly Thr Lys Leu Glu Ile Lys
100 105

<210> 20
<211> 107
<212> PRT
<213> artificial

<220>
<223> c214F8, light chain, VL

<400> 20

Asp Ile Gln Met Thr Gln Thr Thr Ser Ser Leu Ser Ala Ser Leu Gly
1 5 10 15

Asp Arg Val Thr Phe Ser Cys Arg Ala Ser Gln Asp Ile Ser Lys Tyr

```
            20                  25                  30

Leu Asn Trp Tyr Gln Gln Gln Pro Asp Gly Thr Ile Lys Leu Leu Ile
        35                  40                  45

Tyr Tyr Thr Ser Arg Leu His Ser Gly Val Pro Ser Arg Phe Ser Gly
    50                  55                  60

Arg Gly Ser Gly Thr Asp Tyr Ser Leu Thr Ile Thr Asn Leu Glu Gln
65                  70                  75                  80

Glu Asp Ile Ala Thr Tyr Phe Cys Gln Gln Gly Ser Ala Leu Pro Tyr
                85                  90                  95

Thr Phe Gly Gly Gly Thr Lys Leu Glu Ile Lys
            100                 105


<210>  21
<211>  107
<212>  PRT
<213>  artificial

<220>
<223>  c219D6, light chain, VL

<400>  21

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Gln Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Arg Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Ser Thr Leu Pro Tyr
                85                  90                  95

Thr Phe Gly Gly Gly Thr Lys Leu Glu Ile Lys
            100                 105


<210>  22
<211>  107
```

<212> PRT
<213> artificial

<220>
<223> c213B10, light chain, VL

<400> 22

Asp Ile Gln Met Thr Gln Thr Thr Ser Ser Leu Ser Ala Ser Leu Gly
1 5 10 15

Asp Arg Val Thr Ile Ser Cys Arg Ala Ser Gln Asp Ile Ser Lys Tyr
20 25 30

Leu Asn Trp Tyr Gln Gln Gln Pro Asp Gly Thr Ile Lys Leu Leu Ile
35 40 45

Tyr Tyr Thr Ser Arg Leu His Ser Gly Val Pro Ser Arg Phe Ser Gly
50 55 60

Arg Gly Ser Gly Thr Asp Tyr Ser Leu Thr Ile Thr Asn Leu Glu Gln
65 70 75 80

Glu Asp Ile Ala Thr Tyr Phe Cys Gln Gln Gly Ser Ala Leu Pro Tyr
85 90 95

Thr Phe Gly Gly Gly Thr Lys Leu Glu Ile Lys
100 105

<210> 23
<211> 449
<212> PRT
<213> artificial

<220>
<223> c208F2, heavy chain, full length

<400> 23

Gln Val Gln Leu Gln Gln Ser Gly Pro Glu Leu Val Lys Pro Gly Ala
1 5 10 15

Ser Val Lys Met Ser Cys Lys Ala Ser Gly Tyr Thr Phe Thr Ser Tyr
20 25 30

Tyr Ile Tyr Trp Val Lys Gln Arg Pro Gly Gln Gly Leu Glu Trp Leu
35 40 45

Gly Trp Ile Trp Pro Gly Asp Gly Ser Thr Lys Tyr Asn Glu Lys Phe
50 55 60

Lys Asp Lys Thr Thr Leu Thr Ala Asp Lys Ser Ser Asn Thr Ala Tyr

```
65                  70                  75                  80

Met Phe Leu Ser Ser Leu Thr Ser Glu Asp Ser Ala Val Tyr Phe Cys
                85                  90                  95

Ala Ser Pro Met Ile Thr Pro Asn Tyr Ala Met Asp Tyr Trp Gly Gln
            100                 105                 110

Gly Ala Ser Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320
```

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 24
<211> 449
<212> PRT
<213> artificial

<220>
<223> c212A11, heavy chain, full length

<400> 24

Gln Val Gln Leu Gln Gln Ser Gly Pro Glu Leu Val Lys Pro Gly Ala
1 5 10 15

Ser Val Lys Met Ser Cys Lys Ala Ser Gly Tyr Thr Phe Thr Ser Tyr
20 25 30

Tyr Ile His Trp Val Lys Gln Arg Pro Gly Gln Gly Leu Glu Trp Ile
35 40 45

Gly Trp Ile Trp Pro Gly Asp Gly Ser Thr Lys Tyr Asn Glu Lys Phe
50 55 60

Lys Gly Lys Thr Thr Leu Thr Ala Asp Lys Ser Ser Ser Thr Ala Tyr

65 70 75 80

Met Phe Leu Ser Ser Leu Thr Ser Glu Asp Ser Ala Val Tyr Phe Cys
85 90 95

Ala Ser Pro Met Ile Thr Pro Asn Tyr Ala Met Asp Tyr Trp Gly Gln
100 105 110

Gly Ala Ser Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Arg Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 25
<211> 449
<212> PRT
<213> artificial

<220>
<223> c214F8, heavy chain, full length

<400> 25

Gln Val Gln Leu Gln Gln Ser Gly Ser Glu Leu Val Lys Pro Gly Ala
1 5 10 15

Ser Val Lys Leu Ser Cys Lys Ala Ser Gly Tyr Thr Phe Thr Ser Tyr
20 25 30

Tyr Ile His Trp Val Lys Gln Arg Pro Gly Gln Gly Leu Glu Trp Ile
35 40 45

Gly Trp Ile Trp Pro Gly Asp Gly Ser Thr Lys Tyr Asn Glu Arg Phe
50 55 60

Lys Gly Lys Thr Thr Leu Thr Ala Asp Lys Ser Ser Ser Thr Ala Tyr

```
65                  70                  75                  80

Met Phe Leu Ser Ser Leu Thr Ser Glu Asp Ser Ala Val Tyr Phe Cys
                85                  90                  95

Ala Ser Pro Met Ile Thr Pro Asn Tyr Ala Met Asp Tyr Trp Gly Gln
            100                 105                 110

Gly Ala Ser Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320
```

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 26
<211> 449
<212> PRT
<213> artificial

<220>
<223> c219D6, heavy chain, full length

<400> 26

Gln Val Gln Leu Gln Gln Ser Gly Pro Glu Leu Val Lys Pro Gly Asp
1 5 10 15

Ser Val Lys Met Ser Cys Lys Ala Ser Gly Tyr Ser Phe Thr Ser Tyr
20 25 30

Phe Ile His Trp Val Lys Gln Arg Pro Gly Gln Gly Leu Glu Trp Ile
35 40 45

Gly Trp Ile Trp Pro Gly Asp Gly Ser Thr Lys Tyr Asn Glu Lys Phe
50 55 60

Lys Gly Lys Thr Thr Leu Thr Thr Asp Lys Ser Ser Ser Thr Ala Tyr

```
65                  70                  75                  80

Met Phe Leu Asn Ser Leu Thr Ser Glu Asp Ser Ala Val Tyr Phe Cys
                85                  90                  95

Ala Ser Pro Met Ile Thr Pro Asn Tyr Ala Met Asp Tyr Trp Gly Gln
            100                 105                 110

Gly Ala Ser Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320
```

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 27
<211> 449
<212> PRT
<213> artificial

<220>
<223> c213B10, heavy chain, full length

<400> 27

Gln Val Gln Leu Gln Gln Ser Gly Ser Glu Leu Val Lys Pro Gly Ala
1 5 10 15

Ser Val Lys Met Ser Cys Lys Ala Ser Gly Tyr Thr Phe Thr Ser Tyr
20 25 30

Tyr Ile His Trp Val Lys Gln Arg Pro Gly Gln Gly Leu Glu Trp Ile
35 40 45

Gly Trp Ile Trp Pro Gly Asp Gly Ser Thr Lys Tyr Asn Glu Arg Phe
50 55 60

Lys Gly Lys Thr Thr Leu Thr Ala Asp Lys Ser Ser Ser Thr Ala Tyr

```
65                  70                  75                  80

Met Phe Leu Ser Ser Leu Thr Ser Glu Asp Ser Ala Val Tyr Phe Cys
                85                  90                  95

Ala Ser Pro Met Ile Thr Pro Asn Tyr Ala Met Asp Tyr Trp Gly Gln
            100                 105                 110

Gly Ala Ser Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320
```

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 28
<211> 214
<212> PRT
<213> artificial

<220>
<223> c208F2, light chain, full length

<400> 28

Asp Ile Gln Met Thr Gln Thr Thr Ser Ser Leu Ser Ala Ser Leu Gly
1 5 10 15

Asp Arg Val Thr Ile Ser Cys Arg Ala Ser Gln Asp Ile Ser Lys Tyr
20 25 30

Leu Asn Trp Tyr Gln Gln Gln Pro Asp Gly Thr Ile Lys Leu Leu Ile
35 40 45

Tyr Tyr Thr Ser Arg Leu His Ser Gly Val Pro Ser Arg Phe Ser Gly
50 55 60

Arg Gly Ser Gly Thr Asp Tyr Ser Leu Thr Ile Ser Asn Val Glu Gln

65 70 75 80

Glu Asp Ile Ala Thr Tyr Phe Cys Gln Gln Gly Ser Thr Leu Pro Tyr
85 90 95

Thr Phe Gly Gly Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 29
<211> 214
<212> PRT
<213> artificial

<220>
<223> c212A11, light chain, full length

<400> 29

Asp Ile Gln Met Thr Gln Thr Thr Ser Ser Leu Ser Ala Ser Leu Gly
1 5 10 15

Asp Arg Val Thr Ile Ser Cys Arg Ala Ser Gln Asp Ile Asn Lys Tyr
20 25 30

Leu Asn Trp Tyr Gln Gln Gln Pro Asp Gly Thr Val Lys Leu Leu Ile
35 40 45

Tyr Tyr Thr Ser Arg Leu His Ser Gly Val Pro Ser Arg Phe Ser Gly
50 55 60

Arg Gly Ser Gly Thr Asp Tyr Ser Leu Thr Ile Ser Asn Leu Glu Gln
65 70 75 80

Glu Asp Ile Ala Thr Tyr Phe Cys Gln Gln Gly Ser Thr Leu Pro Tyr
85 90 95

Thr Phe Gly Gly Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 30
<211> 214
<212> PRT
<213> artificial

<220>
<223> c214F8, light chain, full length

<400> 30

Asp Ile Gln Met Thr Gln Thr Thr Ser Ser Leu Ser Ala Ser Leu Gly
1 5 10 15

Asp Arg Val Thr Phe Ser Cys Arg Ala Ser Gln Asp Ile Ser Lys Tyr
20 25 30

Leu Asn Trp Tyr Gln Gln Gln Pro Asp Gly Thr Ile Lys Leu Leu Ile
35 40 45

Tyr Tyr Thr Ser Arg Leu His Ser Gly Val Pro Ser Arg Phe Ser Gly
    50                  55                  60

Arg Gly Ser Gly Thr Asp Tyr Ser Leu Thr Ile Thr Asn Leu Glu Gln
65                  70                  75                  80

Glu Asp Ile Ala Thr Tyr Phe Cys Gln Gln Gly Ser Ala Leu Pro Tyr
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 31
<211> 214
<212> PRT
<213> artificial

<220>
<223> c219D6, light chain, full length

<400> 31

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Gln Pro Asp Gly Thr Val Lys Leu Leu Ile
35 40 45

Tyr Tyr Thr Ser Arg Leu His Ser Gly Val Pro Ser Arg Phe Ser Gly
50 55 60

Arg Gly Ser Gly Thr Asp Tyr Ser Leu Thr Ile Ser Asn Leu Glu Gln
65 70 75 80

Glu Asp Ile Ala Thr Tyr Phe Cys Gln Gln Gly Ser Thr Leu Pro Tyr
85 90 95

Thr Phe Gly Gly Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 32
<211> 214
<212> PRT
<213> artificial

<220>
<223> c213B10, light chain, full length

<400> 32

Asp Ile Gln Met Thr Gln Thr Thr Ser Ser Leu Ser Ala Ser Leu Gly
1 5 10 15

Asp Arg Val Thr Ile Ser Cys Arg Ala Ser Gln Asp Ile Ser Lys Tyr

20 25 30

Leu Asn Trp Tyr Gln Gln Gln Pro Asp Gly Thr Ile Lys Leu Leu Ile
35 40 45

Tyr Tyr Thr Ser Arg Leu His Ser Gly Val Pro Ser Arg Phe Ser Gly
50 55 60

Arg Gly Ser Gly Thr Asp Tyr Ser Leu Thr Ile Thr Asn Leu Glu Gln
65 70 75 80

Glu Asp Ile Ala Thr Tyr Phe Cys Gln Gln Gly Ser Ala Leu Pro Tyr
85 90 95

Thr Phe Gly Gly Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 33
<211> 120
<212> PRT
<213> artificial

<220>
<223> hz208F2 (var.1) heavy chain, VH

<400> 33

Gln Val Gln Leu Val Gln Ser Gly Ala Glu Val Lys Lys Pro Gly Ala
1 5 10 15

```
Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Trp Pro Gly Asp Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser
        115                 120


<210>  34
<211>  120
<212>  PRT
<213>  artificial

<220>
<223>  hz208F2 (var. 3), VH

<400>  34

Gln Val Gln Leu Val Gln Ser Gly Ala Glu Val Lys Lys Pro Gly Ala
1               5                   10                  15

Ser Val Lys Met Ser Cys Lys Ala Ser Gly Tyr Thr Phe Thr Ser Tyr
            20                  25                  30

Tyr Ile Tyr Trp Val Lys Gln Ala Pro Gly Gln Gly Leu Glu Trp Leu
        35                  40                  45

Gly Trp Ile Trp Pro Gly Asp Gly Ser Thr Lys Tyr Asn Glu Lys Phe
    50                  55                  60

Gln Gly Arg Val Thr Leu Thr Ala Asp Lys Ser Ser Asn Thr Ala Tyr
65                  70                  75                  80

Met Phe Leu Ser Ser Leu Arg Ser Glu Asp Thr Ala Val Tyr Phe Cys
                85                  90                  95
```

```
Ala Ser Pro Met Ile Thr Pro Asn Tyr Ala Met Asp Tyr Trp Gly Gln
            100                 105                 110

Gly Thr Leu Val Thr Val Ser Ser
        115                 120


<210>  35
<211>  107
<212>  PRT
<213>  artificial

<220>
<223>  hz208F2 (var. 1), VL

<400>  35

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Lys Tyr
            20                  25                  30

Leu Asn Trp Tyr Gln Gln Lys Pro Gly Lys Ala Pro Lys Leu Leu Ile
        35                  40                  45

Tyr Tyr Thr Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Ser Thr Leu Pro Tyr
                85                  90                  95

Thr Phe Gly Gly Gly Thr Lys Val Glu Ile Lys
            100                 105


<210>  36
<211>  107
<212>  PRT
<213>  artificial

<220>
<223>  hz208F2 (var.3), VL

<400>  36

Asp Ile Gln Met Thr Gln Ser Pro Ser Ser Leu Ser Ala Ser Val Gly
1               5                   10                  15

Asp Arg Val Thr Ile Ser Cys Arg Ala Ser Gln Asp Ile Ser Lys Tyr
            20                  25                  30
```

```
Leu Asn Trp Tyr Gln Gln Lys Pro Gly Lys Ala Pro Lys Leu Leu Ile
        35                  40                  45

Tyr Tyr Thr Ser Arg Leu His Ser Gly Val Pro Ser Arg Phe Ser Gly
    50                  55                  60

Arg Gly Ser Gly Thr Asp Tyr Ser Leu Thr Ile Ser Asn Leu Gln Pro
65                  70                  75                  80

Glu Asp Phe Ala Thr Tyr Phe Cys Gln Gln Gly Ser Thr Leu Pro Tyr
                85                  90                  95

Thr Phe Gly Gly Gly Thr Lys Val Glu Ile Lys
            100                 105


<210>  37
<211>  449
<212>  PRT
<213>  artificial

<220>
<223>  hz208F2 (var. 1), heavy chain, full length

<400>  37

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Trp Pro Gly Asp Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125
```

```
Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380
```

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 38
<211> 449
<212> PRT
<213> artificial

<220>
<223> hz208F2 (var.3), heavy chain full length

<400> 38

Gln Val Gln Leu Val Gln Ser Gly Ala Glu Val Lys Lys Pro Gly Ala
1 5 10 15

Ser Val Lys Met Ser Cys Lys Ala Ser Gly Tyr Thr Phe Thr Ser Tyr
20 25 30

Tyr Ile Tyr Trp Val Lys Gln Ala Pro Gly Gln Gly Leu Glu Trp Leu
35 40 45

Gly Trp Ile Trp Pro Gly Asp Gly Ser Thr Lys Tyr Asn Glu Lys Phe
50 55 60

Gln Gly Arg Val Thr Leu Thr Ala Asp Lys Ser Ser Asn Thr Ala Tyr
65 70 75 80

Met Phe Leu Ser Ser Leu Arg Ser Glu Asp Thr Ala Val Tyr Phe Cys
85 90 95

Ala Ser Pro Met Ile Thr Pro Asn Tyr Ala Met Asp Tyr Trp Gly Gln
100 105 110

Gly Thr Leu Val Thr Val Ser Ser Ala Ser Thr Lys Gly Pro Ser Val
115 120 125

```
Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380
```

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 39
<211> 214
<212> PRT
<213> artificial

<220>
<223> hz208F2 (var. 1), light chain, full length

<400> 39

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Lys Tyr
20 25 30

Leu Asn Trp Tyr Gln Gln Lys Pro Gly Lys Ala Pro Lys Leu Leu Ile
35 40 45

Tyr Tyr Thr Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Gly Ser Thr Leu Pro Tyr
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 40
<211> 214
<212> PRT
<213> artificial

<220>
<223> hz208F2 (var.3), light chain, full length

<400> 40

Asp Ile Gln Met Thr Gln Ser Pro Ser Ser Leu Ser Ala Ser Val Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu His Ser Gly Val Pro Ser Arg Phe Ser Gly
    50                  55                  60

Arg Gly Ser Gly Thr Asp Tyr Ser Leu Thr Ile Ser Asn Leu Gln Pro
65                  70                  75                  80

Glu Asp Phe Ala Thr Tyr Phe Cys Gln Gln Gly Ser Thr Leu Pro Tyr
                85                  90                  95

Thr Phe Gly Gly Gly Thr Lys Val Glu Ile Lys Arg Thr Val Ala Ala
            100                 105                 110

```
Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  41
<211>  120
<212>  PRT
<213>  artificial

<220>
<223>  hz208F2 (var.2) heavy chain, VH


<220>
<221>  MISC_FEATURE
<222>  (20)..(20)
<223>  Met may be replaced by Val

<220>
<221>  MISC_FEATURE
<222>  (34)..(34)
<223>  Ile may be replaced by Met

<220>
<221>  MISC_FEATURE
<222>  (35)..(35)
<223>  Tyr may be replaced by His

<220>
<221>  MISC_FEATURE
<222>  (38)..(38)
<223>  Lys may be replaced by Arg

<220>
<221>  MISC_FEATURE
<222>  (48)..(48)
<223>  Leu may be replaced by Met

<220>
```

<221> MISC_FEATURE
<222> (50)..(50)
<223> Trp may be replaced by Ile

<220>
<221> MISC_FEATURE
<222> (59)..(59)
<223> Lys may be replaced by Ser

<220>
<221> MISC_FEATURE
<222> (61)..(61)
<223> Asn may be replaced by Ala

<220>
<221> MISC_FEATURE
<222> (62)..(62)
<223> Glu may be replaced by Gln

<220>
<221> MISC_FEATURE
<222> (70)..(70)
<223> Leu may be replaced by Met

<220>
<221> MISC_FEATURE
<222> (72)..(72)
<223> Ala may be replaced by Arg

<220>
<221> MISC_FEATURE
<222> (74)..(74)
<223> Lys may be replaced by Thr

<220>
<221> MISC_FEATURE
<222> (76)..(76)
<223> Ser may be replaced by Thr

<220>
<221> MISC_FEATURE
<222> (77)..(77)
<223> Asn may be replaced by Ser

<220>
<221> MISC_FEATURE
<222> (79)..(79)
<223> Ala may be replaced by Val

<220>
<221> MISC_FEATURE
<222> (82)..(82)
<223> Phe may be replaced by Glu

<220>
<221> MISC_FEATURE
<222> (95)..(95)
<223> Phe may be replaced by Tyr

<400> 41

Gln Val Gln Leu Val Gln Ser Gly Ala Glu Val Lys Lys Pro Gly Ala
1               5                   10                  15

Ser Val Lys Met Ser Cys Lys Ala Ser Gly Tyr Thr Phe Thr Ser Tyr
20 25 30

Tyr Ile Tyr Trp Val Lys Gln Ala Pro Gly Gln Gly Leu Glu Trp Leu
35 40 45

Gly Trp Ile Trp Pro Gly Asp Gly Ser Thr Lys Tyr Asn Glu Lys Phe
50 55 60

Gln Gly Arg Val Thr Leu Thr Ala Asp Lys Ser Ser Asn Thr Ala Tyr
65 70 75 80

Met Phe Leu Ser Ser Leu Arg Ser Glu Asp Thr Ala Val Tyr Phe Cys
85 90 95

Ala Ser Pro Met Ile Thr Pro Asn Tyr Ala Met Asp Tyr Trp Gly Gln
100 105 110

Gly Thr Leu Val Thr Val Ser Ser
115 120

<210> 42
<211> 107
<212> PRT
<213> artificial

<220>
<223> hz208F2 (var. 2), light chain, VL

<220>
<221> MISC_FEATURE
<222> (22)..(22)
<223> Ser may be replaced by Thr

<220>
<221> MISC_FEATURE
<222> (53)..(53)
<223> Arg may be replaced by Ser

<220>
<221> MISC_FEATURE
<222> (55)..(55)
<223> His may be replaced by Gln

<220>
<221> MISC_FEATURE
<222> (65)..(65)
<223> Arg may be replaced by Ser

<220>
<221> MISC_FEATURE
<222> (71)..(71)
<223> Tyr may be replaced by Phe

<220>
<221> MISC_FEATURE
<222> (72)..(72)
<223> Ser may be replaced by Thr

<220>
<221> MISC_FEATURE
<222> (77)..(77)
<223> Asn may be replaced by Ser

<220>
<221> MISC_FEATURE
<222> (87)..(87)
<223> Phe may be replaced by Tyr

<400> 42

```
Asp Ile Gln Met Thr Gln Ser Pro Ser Ser Leu Ser Ala Ser Val Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu His Ser Gly Val Pro Ser Arg Phe Ser Gly
    50                  55                  60

Arg Gly Ser Gly Thr Asp Tyr Ser Leu Thr Ile Ser Asn Leu Gln Pro
65                  70                  75                  80

Glu Asp Phe Ala Thr Tyr Phe Cys Gln Gln Gly Ser Thr Leu Pro Tyr
                85                  90                  95

Thr Phe Gly Gly Gly Thr Lys Val Glu Ile Lys
            100                 105
```

<210> 43
<211> 329
<212> PRT
<213> artificial

<220>
<223> Constant domain (VH) IgG1

<400> 43

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30
```

```
Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285
```

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210> 44
<211> 326
<212> PRT
<213> artificial

<220>
<223> Constant domain (VH) IgG4 (S228P)

<400> 44

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

```
Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly
                325


<210>  45
<211>  107
<212>  PRT
<213>  artificial

<220>
<223>  Domain kappa (VL)

<400>  45

Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30
```

Tyr Pro Arg Glu Ala Lys Val Gln Trp Lys Val Asp Asn Ala Leu Gln
35 40 45

Ser Gly Asn Ser Gln Glu Ser Val Thr Glu Gln Asp Ser Lys Asp Ser
50 55 60

Thr Tyr Ser Leu Ser Ser Thr Leu Thr Leu Ser Lys Ala Asp Tyr Glu
65 70 75 80

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 46
<211> 98
<212> PRT
<213> artificial

<220>
<223> Human germline IGHV1-46*01

<400> 46

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

Gly Ile Ile Asn Pro Ser Gly Gly Ser Thr Ser Tyr Ala Gln Lys Phe
50 55 60

Gln Gly Arg Val Thr Met Thr Arg Asp Thr Ser Thr Ser Thr Val Tyr
65 70 75 80

Met Glu Leu Ser Ser Leu Arg Ser Glu Asp Thr Ala Val Tyr Tyr Cys
85 90 95

Ala Arg

<210> 47
<211> 95
<212> PRT
<213> artificial

<220>
<223> Human germline IGKV1-39*01

<400> 47

Asp Ile Gln Met Thr Gln Ser Pro Ser Ser Leu Ser Ala Ser Val Gly
1 5 10 15

Asp Arg Val Thr Ile Thr Cys Arg Ala Ser Gln Ser Ile Ser Ser Tyr
20 25 30

Leu Asn Trp Tyr Gln Gln Lys Pro Gly Lys Ala Pro Lys Leu Leu Ile
35 40 45

Tyr Ala Ala Ser Ser Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Ser Tyr Ser Thr Pro
85 90 95

<210> 48
<211> 15
<212> PRT
<213> artificial

<220>
<223> Human germline IGHJ4*01

<400> 48

Tyr Phe Asp Tyr Trp Gly Gln Gly Thr Leu Val Thr Val Ser Ser
1 5 10 15

<210> 49
<211> 12
<212> PRT
<213> artificial

<220>
<223> Human germline IGKJ4*01

<400> 49

Leu Thr Phe Gly Gly Gly Thr Lys Val Glu Ile Lys
1 5 10

<210> 50
<211> 1367
<212> PRT
<213> artificial

<220>

<223> IGF-1R (human)

<400> 50

Met Lys Ser Gly Ser Gly Gly Gly Ser Pro Thr Ser Leu Trp Gly Leu
1               5                   10                  15

Leu Phe Leu Ser Ala Ala Leu Ser Leu Trp Pro Thr Ser Gly Glu Ile
            20                  25                  30

Cys Gly Pro Gly Ile Asp Ile Arg Asn Asp Tyr Gln Gln Leu Lys Arg
        35                  40                  45

Leu Glu Asn Cys Thr Val Ile Glu Gly Tyr Leu His Ile Leu Leu Ile
    50                  55                  60

Ser Lys Ala Glu Asp Tyr Arg Ser Tyr Arg Phe Pro Lys Leu Thr Val
65                  70                  75                  80

Ile Thr Glu Tyr Leu Leu Leu Phe Arg Val Ala Gly Leu Glu Ser Leu
                85                  90                  95

Gly Asp Leu Phe Pro Asn Leu Thr Val Ile Arg Gly Trp Lys Leu Phe
            100                 105                 110

Tyr Asn Tyr Ala Leu Val Ile Phe Glu Met Thr Asn Leu Lys Asp Ile
        115                 120                 125

Gly Leu Tyr Asn Leu Arg Asn Ile Thr Arg Gly Ala Ile Arg Ile Glu
    130                 135                 140

Lys Asn Ala Asp Leu Cys Tyr Leu Ser Thr Val Asp Trp Ser Leu Ile
145                 150                 155                 160

Leu Asp Ala Val Ser Asn Asn Tyr Ile Val Gly Asn Lys Pro Pro Lys
                165                 170                 175

Glu Cys Gly Asp Leu Cys Pro Gly Thr Met Glu Glu Lys Pro Met Cys
            180                 185                 190

Glu Lys Thr Thr Ile Asn Asn Glu Tyr Asn Tyr Arg Cys Trp Thr Thr
        195                 200                 205

Asn Arg Cys Gln Lys Met Cys Pro Ser Thr Cys Gly Lys Arg Ala Cys
    210                 215                 220

Thr Glu Asn Asn Glu Cys Cys His Pro Glu Cys Leu Gly Ser Cys Ser
225                 230                 235                 240

```
Ala Pro Asp Asn Asp Thr Ala Cys Val Ala Cys Arg His Tyr Tyr Tyr
                245                 250                 255

Ala Gly Val Cys Val Pro Ala Cys Pro Pro Asn Thr Tyr Arg Phe Glu
            260                 265                 270

Gly Trp Arg Cys Val Asp Arg Asp Phe Cys Ala Asn Ile Leu Ser Ala
        275                 280                 285

Glu Ser Ser Asp Ser Glu Gly Phe Val Ile His Asp Gly Glu Cys Met
    290                 295                 300

Gln Glu Cys Pro Ser Gly Phe Ile Arg Asn Gly Ser Gln Ser Met Tyr
305                 310                 315                 320

Cys Ile Pro Cys Glu Gly Pro Cys Pro Lys Val Cys Glu Glu Glu Lys
                325                 330                 335

Lys Thr Lys Thr Ile Asp Ser Val Thr Ser Ala Gln Met Leu Gln Gly
            340                 345                 350

Cys Thr Ile Phe Lys Gly Asn Leu Leu Ile Asn Ile Arg Arg Gly Asn
        355                 360                 365

Asn Ile Ala Ser Glu Leu Glu Asn Phe Met Gly Leu Ile Glu Val Val
    370                 375                 380

Thr Gly Tyr Val Lys Ile Arg His Ser His Ala Leu Val Ser Leu Ser
385                 390                 395                 400

Phe Leu Lys Asn Leu Arg Leu Ile Leu Gly Glu Glu Gln Leu Glu Gly
                405                 410                 415

Asn Tyr Ser Phe Tyr Val Leu Asp Asn Gln Asn Leu Gln Gln Leu Trp
            420                 425                 430

Asp Trp Asp His Arg Asn Leu Thr Ile Lys Ala Gly Lys Met Tyr Phe
        435                 440                 445

Ala Phe Asn Pro Lys Leu Cys Val Ser Glu Ile Tyr Arg Met Glu Glu
    450                 455                 460

Val Thr Gly Thr Lys Gly Arg Gln Ser Lys Gly Asp Ile Asn Thr Arg
465                 470                 475                 480

Asn Asn Gly Glu Arg Ala Ser Cys Glu Ser Asp Val Leu His Phe Thr
                485                 490                 495
```

```
Ser Thr Thr Thr Ser Lys Asn Arg Ile Ile Ile Thr Trp His Arg Tyr
            500                 505                 510

Arg Pro Pro Asp Tyr Arg Asp Leu Ile Ser Phe Thr Val Tyr Tyr Lys
        515                 520                 525

Glu Ala Pro Phe Lys Asn Val Thr Glu Tyr Asp Gly Gln Asp Ala Cys
    530                 535                 540

Gly Ser Asn Ser Trp Asn Met Val Asp Val Asp Leu Pro Pro Asn Lys
545                 550                 555                 560

Asp Val Glu Pro Gly Ile Leu Leu His Gly Leu Lys Pro Trp Thr Gln
                565                 570                 575

Tyr Ala Val Tyr Val Lys Ala Val Thr Leu Thr Met Val Glu Asn Asp
            580                 585                 590

His Ile Arg Gly Ala Lys Ser Glu Ile Leu Tyr Ile Arg Thr Asn Ala
        595                 600                 605

Ser Val Pro Ser Ile Pro Leu Asp Val Leu Ser Ala Ser Asn Ser Ser
    610                 615                 620

Ser Gln Leu Ile Val Lys Trp Asn Pro Pro Ser Leu Pro Asn Gly Asn
625                 630                 635                 640

Leu Ser Tyr Tyr Ile Val Arg Trp Gln Arg Gln Pro Gln Asp Gly Tyr
                645                 650                 655

Leu Tyr Arg His Asn Tyr Cys Ser Lys Asp Lys Ile Pro Ile Arg Lys
            660                 665                 670

Tyr Ala Asp Gly Thr Ile Asp Ile Glu Glu Val Thr Glu Asn Pro Lys
        675                 680                 685

Thr Glu Val Cys Gly Gly Glu Lys Gly Pro Cys Cys Ala Cys Pro Lys
    690                 695                 700

Thr Glu Ala Glu Lys Gln Ala Glu Lys Glu Glu Ala Glu Tyr Arg Lys
705                 710                 715                 720

Val Phe Glu Asn Phe Leu His Asn Ser Ile Phe Val Pro Arg Pro Glu
                725                 730                 735

Arg Lys Arg Arg Asp Val Met Gln Val Ala Asn Thr Thr Met Ser Ser
```

```
            740                 745                 750

Arg Ser Arg Asn Thr Thr Ala Ala Asp Thr Tyr Asn Ile Thr Asp Pro
        755                 760                 765

Glu Glu Leu Glu Thr Glu Tyr Pro Phe Phe Glu Ser Arg Val Asp Asn
    770                 775                 780

Lys Glu Arg Thr Val Ile Ser Asn Leu Arg Pro Phe Thr Leu Tyr Arg
785                 790                 795                 800

Ile Asp Ile His Ser Cys Asn His Glu Ala Glu Lys Leu Gly Cys Ser
                805                 810                 815

Ala Ser Asn Phe Val Phe Ala Arg Thr Met Pro Ala Glu Gly Ala Asp
            820                 825                 830

Asp Ile Pro Gly Pro Val Thr Trp Glu Pro Arg Pro Glu Asn Ser Ile
        835                 840                 845

Phe Leu Lys Trp Pro Glu Pro Glu Asn Pro Asn Gly Leu Ile Leu Met
    850                 855                 860

Tyr Glu Ile Lys Tyr Gly Ser Gln Val Glu Asp Gln Arg Glu Cys Val
865                 870                 875                 880

Ser Arg Gln Glu Tyr Arg Lys Tyr Gly Gly Ala Lys Leu Asn Arg Leu
                885                 890                 895

Asn Pro Gly Asn Tyr Thr Ala Arg Ile Gln Ala Thr Ser Leu Ser Gly
            900                 905                 910

Asn Gly Ser Trp Thr Asp Pro Val Phe Phe Tyr Val Gln Ala Lys Thr
        915                 920                 925

Gly Tyr Glu Asn Phe Ile His Leu Ile Ile Ala Leu Pro Val Ala Val
    930                 935                 940

Leu Leu Ile Val Gly Gly Leu Val Ile Met Leu Tyr Val Phe His Arg
945                 950                 955                 960

Lys Arg Asn Asn Ser Arg Leu Gly Asn Gly Val Leu Tyr Ala Ser Val
                965                 970                 975

Asn Pro Glu Tyr Phe Ser Ala Ala Asp Val Tyr Val Pro Asp Glu Trp
            980                 985                 990
```

```
Glu Val Ala Arg Glu Lys Ile Thr  Met Ser Arg Glu Leu  Gly Gln Gly
        995                 1000                 1005

Ser Phe  Gly Met Val Tyr Glu  Gly Val Ala Lys Gly  Val Val Lys
    1010                 1015                 1020

Asp Glu  Pro Glu Thr Arg Val  Ala Ile Lys Thr Val  Asn Glu Ala
    1025                 1030                 1035

Ala Ser  Met Arg Glu Arg Ile  Glu Phe Leu Asn Glu  Ala Ser Val
    1040                 1045                 1050

Met Lys  Glu Phe Asn Cys His  His Val Val Arg Leu  Leu Gly Val
    1055                 1060                 1065

Val Ser  Gln Gly Gln Pro Thr  Leu Val Ile Met Glu  Leu Met Thr
    1070                 1075                 1080

Arg Gly  Asp Leu Lys Ser Tyr  Leu Arg Ser Leu Arg  Pro Glu Met
    1085                 1090                 1095

Glu Asn  Asn Pro Val Leu Ala  Pro Pro Ser Leu Ser  Lys Met Ile
    1100                 1105                 1110

Gln Met  Ala Gly Glu Ile Ala  Asp Gly Met Ala Tyr  Leu Asn Ala
    1115                 1120                 1125

Asn Lys  Phe Val His Arg Asp  Leu Ala Ala Arg Asn  Cys Met Val
    1130                 1135                 1140

Ala Glu  Asp Phe Thr Val Lys  Ile Gly Asp Phe Gly  Met Thr Arg
    1145                 1150                 1155

Asp Ile  Tyr Glu Thr Asp Tyr  Tyr Arg Lys Gly Gly  Lys Gly Leu
    1160                 1165                 1170

Leu Pro  Val Arg Trp Met Ser  Pro Glu Ser Leu Lys  Asp Gly Val
    1175                 1180                 1185

Phe Thr  Thr Tyr Ser Asp Val  Trp Ser Phe Gly Val  Val Leu Trp
    1190                 1195                 1200

Glu Ile  Ala Thr Leu Ala Glu  Gln Pro Tyr Gln Gly  Leu Ser Asn
    1205                 1210                 1215

Glu Gln  Val Leu Arg Phe Val  Met Glu Gly Gly Leu  Leu Asp Lys
    1220                 1225                 1230
```

Pro Asp Asn Cys Pro Asp Met Leu Phe Glu Leu Met Arg Met Cys
    1235                1240                1245

Trp Gln Tyr Asn Pro Lys Met Arg Pro Ser Phe Leu Glu Ile Ile
    1250                1255                1260

Ser Ser Ile Lys Glu Glu Met Glu Pro Gly Phe Arg Glu Val Ser
    1265                1270                1275

Phe Tyr Tyr Ser Glu Glu Asn Lys Leu Pro Glu Pro Glu Glu Leu
    1280                1285                1290

Asp Leu Glu Pro Glu Asn Met Glu Ser Val Pro Leu Asp Pro Ser
    1295                1300                1305

Ala Ser Ser Ser Ser Leu Pro Leu Pro Asp Arg His Ser Gly His
    1310                1315                1320

Lys Ala Glu Asn Gly Pro Gly Pro Gly Val Leu Val Leu Arg Ala
    1325                1330                1335

Ser Phe Asp Glu Arg Gln Pro Tyr Ala His Met Asn Gly Gly Arg
    1340                1345                1350

Lys Asn Glu Arg Ala Leu Pro Leu Pro Gln Ser Ser Thr Cys
    1355                1360                1365

<210> 51
<211> 932
<212> PRT
<213> artificial

<220>
<223> IGF-1R ECD (human)

<400> 51

Met Lys Ser Gly Ser Gly Gly Gly Ser Pro Thr Ser Leu Trp Gly Leu
1               5                   10                  15

Leu Phe Leu Ser Ala Ala Leu Ser Leu Trp Pro Thr Ser Gly Glu Ile
            20                  25                  30

Cys Gly Pro Gly Ile Asp Ile Arg Asn Asp Tyr Gln Gln Leu Lys Arg
        35                  40                  45

Leu Glu Asn Cys Thr Val Ile Glu Gly Tyr Leu His Ile Leu Leu Ile
    50                  55                  60

```
Ser Lys Ala Glu Asp Tyr Arg Ser Tyr Arg Phe Pro Lys Leu Thr Val
65                  70                  75                  80

Ile Thr Glu Tyr Leu Leu Leu Phe Arg Val Ala Gly Leu Glu Ser Leu
                85                  90                  95

Gly Asp Leu Phe Pro Asn Leu Thr Val Ile Arg Gly Trp Lys Leu Phe
            100                 105                 110

Tyr Asn Tyr Ala Leu Val Ile Phe Glu Met Thr Asn Leu Lys Asp Ile
        115                 120                 125

Gly Leu Tyr Asn Leu Arg Asn Ile Thr Arg Gly Ala Ile Arg Ile Glu
    130                 135                 140

Lys Asn Ala Asp Leu Cys Tyr Leu Ser Thr Val Asp Trp Ser Leu Ile
145                 150                 155                 160

Leu Asp Ala Val Ser Asn Asn Tyr Ile Val Gly Asn Lys Pro Pro Lys
                165                 170                 175

Glu Cys Gly Asp Leu Cys Pro Gly Thr Met Glu Glu Lys Pro Met Cys
            180                 185                 190

Glu Lys Thr Thr Ile Asn Asn Glu Tyr Asn Tyr Arg Cys Trp Thr Thr
        195                 200                 205

Asn Arg Cys Gln Lys Met Cys Pro Ser Thr Cys Gly Lys Arg Ala Cys
    210                 215                 220

Thr Glu Asn Asn Glu Cys Cys His Pro Glu Cys Leu Gly Ser Cys Ser
225                 230                 235                 240

Ala Pro Asp Asn Asp Thr Ala Cys Val Ala Cys Arg His Tyr Tyr Tyr
                245                 250                 255

Ala Gly Val Cys Val Pro Ala Cys Pro Pro Asn Thr Tyr Arg Phe Glu
            260                 265                 270

Gly Trp Arg Cys Val Asp Arg Asp Phe Cys Ala Asn Ile Leu Ser Ala
        275                 280                 285

Glu Ser Ser Asp Ser Glu Gly Phe Val Ile His Asp Gly Glu Cys Met
    290                 295                 300

Gln Glu Cys Pro Ser Gly Phe Ile Arg Asn Gly Ser Gln Ser Met Tyr
305                 310                 315                 320
```

```
Cys Ile Pro Cys Glu Gly Pro Cys Pro Lys Val Cys Glu Glu Glu Lys
                325                 330                 335

Lys Thr Lys Thr Ile Asp Ser Val Thr Ser Ala Gln Met Leu Gln Gly
            340                 345                 350

Cys Thr Ile Phe Lys Gly Asn Leu Leu Ile Asn Ile Arg Arg Gly Asn
        355                 360                 365

Asn Ile Ala Ser Glu Leu Glu Asn Phe Met Gly Leu Ile Glu Val Val
    370                 375                 380

Thr Gly Tyr Val Lys Ile Arg His Ser His Ala Leu Val Ser Leu Ser
385                 390                 395                 400

Phe Leu Lys Asn Leu Arg Leu Ile Leu Gly Glu Glu Gln Leu Glu Gly
                405                 410                 415

Asn Tyr Ser Phe Tyr Val Leu Asp Asn Gln Asn Leu Gln Gln Leu Trp
            420                 425                 430

Asp Trp Asp His Arg Asn Leu Thr Ile Lys Ala Gly Lys Met Tyr Phe
        435                 440                 445

Ala Phe Asn Pro Lys Leu Cys Val Ser Glu Ile Tyr Arg Met Glu Glu
    450                 455                 460

Val Thr Gly Thr Lys Gly Arg Gln Ser Lys Gly Asp Ile Asn Thr Arg
465                 470                 475                 480

Asn Asn Gly Glu Arg Ala Ser Cys Glu Ser Asp Val Leu His Phe Thr
                485                 490                 495

Ser Thr Thr Thr Ser Lys Asn Arg Ile Ile Ile Thr Trp His Arg Tyr
            500                 505                 510

Arg Pro Pro Asp Tyr Arg Asp Leu Ile Ser Phe Thr Val Tyr Tyr Lys
        515                 520                 525

Glu Ala Pro Phe Lys Asn Val Thr Glu Tyr Asp Gly Gln Asp Ala Cys
    530                 535                 540

Gly Ser Asn Ser Trp Asn Met Val Asp Val Asp Leu Pro Pro Asn Lys
545                 550                 555                 560

Asp Val Glu Pro Gly Ile Leu Leu His Gly Leu Lys Pro Trp Thr Gln
                565                 570                 575
```

Tyr Ala Val Tyr Val Lys Ala Val Thr Leu Thr Met Val Glu Asn Asp
580 585 590

His Ile Arg Gly Ala Lys Ser Glu Ile Leu Tyr Ile Arg Thr Asn Ala
595 600 605

Ser Val Pro Ser Ile Pro Leu Asp Val Leu Ser Ala Ser Asn Ser Ser
610 615 620

Ser Gln Leu Ile Val Lys Trp Asn Pro Pro Ser Leu Pro Asn Gly Asn
625 630 635 640

Leu Ser Tyr Tyr Ile Val Arg Trp Gln Arg Gln Pro Gln Asp Gly Tyr
645 650 655

Leu Tyr Arg His Asn Tyr Cys Ser Lys Asp Lys Ile Pro Ile Arg Lys
660 665 670

Tyr Ala Asp Gly Thr Ile Asp Ile Glu Glu Val Thr Glu Asn Pro Lys
675 680 685

Thr Glu Val Cys Gly Gly Glu Lys Gly Pro Cys Cys Ala Cys Pro Lys
690 695 700

Thr Glu Ala Glu Lys Gln Ala Glu Lys Glu Glu Ala Glu Tyr Arg Lys
705 710 715 720

Val Phe Glu Asn Phe Leu His Asn Ser Ile Phe Val Pro Arg Pro Glu
725 730 735

Arg Lys Arg Arg Asp Val Met Gln Val Ala Asn Thr Thr Met Ser Ser
740 745 750

Arg Ser Arg Asn Thr Thr Ala Ala Asp Thr Tyr Asn Ile Thr Asp Pro
755 760 765

Glu Glu Leu Glu Thr Glu Tyr Pro Phe Phe Glu Ser Arg Val Asp Asn
770 775 780

Lys Glu Arg Thr Val Ile Ser Asn Leu Arg Pro Phe Thr Leu Tyr Arg
785 790 795 800

Ile Asp Ile His Ser Cys Asn His Glu Ala Glu Lys Leu Gly Cys Ser
805 810 815

Ala Ser Asn Phe Val Phe Ala Arg Thr Met Pro Ala Glu Gly Ala Asp

820 825 830

Asp Ile Pro Gly Pro Val Thr Trp Glu Pro Arg Pro Glu Asn Ser Ile
835 840 845

Phe Leu Lys Trp Pro Glu Pro Glu Asn Pro Asn Gly Leu Ile Leu Met
850 855 860

Tyr Glu Ile Lys Tyr Gly Ser Gln Val Glu Asp Gln Arg Glu Cys Val
865 870 875 880

Ser Arg Gln Glu Tyr Arg Lys Tyr Gly Gly Ala Lys Leu Asn Arg Leu
885 890 895

Asn Pro Gly Asn Tyr Thr Ala Arg Ile Gln Ala Thr Ser Leu Ser Gly
900 905 910

Asn Gly Ser Trp Thr Asp Pro Val Phe Phe Tyr Val Gln Ala Lys Thr
915 920 925

Gly Tyr Glu Asn
930

<210> 52
<211> 512
<212> PRT
<213> artificial

<220>
<223> IGF-1R ECD Nterminal (human)

<400> 52

Met Lys Ser Gly Ser Gly Gly Gly Ser Pro Thr Ser Leu Trp Gly Leu
1 5 10 15

Leu Phe Leu Ser Ala Ala Leu Ser Leu Trp Pro Thr Ser Gly Glu Ile
20 25 30

Cys Gly Pro Gly Ile Asp Ile Arg Asn Asp Tyr Gln Gln Leu Lys Arg
35 40 45

Leu Glu Asn Cys Thr Val Ile Glu Gly Tyr Leu His Ile Leu Leu Ile
50 55 60

Ser Lys Ala Glu Asp Tyr Arg Ser Tyr Arg Phe Pro Lys Leu Thr Val
65 70 75 80

Ile Thr Glu Tyr Leu Leu Leu Phe Arg Val Ala Gly Leu Glu Ser Leu
85 90 95

```
Gly Asp Leu Phe Pro Asn Leu Thr Val Ile Arg Gly Trp Lys Leu Phe
            100                 105                 110

Tyr Asn Tyr Ala Leu Val Ile Phe Glu Met Thr Asn Leu Lys Asp Ile
        115                 120                 125

Gly Leu Tyr Asn Leu Arg Asn Ile Thr Arg Gly Ala Ile Arg Ile Glu
    130                 135                 140

Lys Asn Ala Asp Leu Cys Tyr Leu Ser Thr Val Asp Trp Ser Leu Ile
145                 150                 155                 160

Leu Asp Ala Val Ser Asn Asn Tyr Ile Val Gly Asn Lys Pro Pro Lys
                165                 170                 175

Glu Cys Gly Asp Leu Cys Pro Gly Thr Met Glu Glu Lys Pro Met Cys
            180                 185                 190

Glu Lys Thr Thr Ile Asn Asn Glu Tyr Asn Tyr Arg Cys Trp Thr Thr
        195                 200                 205

Asn Arg Cys Gln Lys Met Cys Pro Ser Thr Cys Gly Lys Arg Ala Cys
    210                 215                 220

Thr Glu Asn Asn Glu Cys Cys His Pro Glu Cys Leu Gly Ser Cys Ser
225                 230                 235                 240

Ala Pro Asp Asn Asp Thr Ala Cys Val Ala Cys Arg His Tyr Tyr Tyr
                245                 250                 255

Ala Gly Val Cys Val Pro Ala Cys Pro Pro Asn Thr Tyr Arg Phe Glu
            260                 265                 270

Gly Trp Arg Cys Val Asp Arg Asp Phe Cys Ala Asn Ile Leu Ser Ala
        275                 280                 285

Glu Ser Ser Asp Ser Glu Gly Phe Val Ile His Asp Gly Glu Cys Met
    290                 295                 300

Gln Glu Cys Pro Ser Gly Phe Ile Arg Asn Gly Ser Gln Ser Met Tyr
305                 310                 315                 320

Cys Ile Pro Cys Glu Gly Pro Cys Pro Lys Val Cys Glu Glu Glu Lys
                325                 330                 335

Lys Thr Lys Thr Ile Asp Ser Val Thr Ser Ala Gln Met Leu Gln Gly
```

340 345 350

Cys Thr Ile Phe Lys Gly Asn Leu Leu Ile Asn Ile Arg Arg Gly Asn
355 360 365

Asn Ile Ala Ser Glu Leu Glu Asn Phe Met Gly Leu Ile Glu Val Val
370 375 380

Thr Gly Tyr Val Lys Ile Arg His Ser His Ala Leu Val Ser Leu Ser
385 390 395 400

Phe Leu Lys Asn Leu Arg Leu Ile Leu Gly Glu Glu Gln Leu Glu Gly
405 410 415

Asn Tyr Ser Phe Tyr Val Leu Asp Asn Gln Asn Leu Gln Gln Leu Trp
420 425 430

Asp Trp Asp His Arg Asn Leu Thr Ile Lys Ala Gly Lys Met Tyr Phe
435 440 445

Ala Phe Asn Pro Lys Leu Cys Val Ser Glu Ile Tyr Arg Met Glu Glu
450 455 460

Val Thr Gly Thr Lys Gly Arg Gln Ser Lys Gly Asp Ile Asn Thr Arg
465 470 475 480

Asn Asn Gly Glu Arg Ala Ser Cys Glu Ser Asp Val Leu His Phe Thr
485 490 495

Ser Thr Thr Thr Ser Lys Asn Arg Ile Ile Ile Thr Trp His Arg Tyr
500 505 510

<210> 53
<211> 449
<212> PRT
<213> artificial

<220>
<223> hz208F2 heavy chain H076 full length

<400> 53

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Ile His Trp Val Arg Gln Ala Pro Gly Gln Gly Leu Glu Trp Met
35 40 45

```
Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
```

290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 54
<211> 120
<212> PRT
<213> artificial

<220>
<223> hz208F2 heavy chain H077, VH

<400> 54

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Ile His Trp Val Arg Gln Ala Pro Gly Gln Gly Leu Glu Trp Met
35 40 45

```
Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Phe Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser
        115                 120


<210>  55
<211>  449
<212>  PRT
<213>  artificial

<220>
<223>  hz208F2 heavy chain H077 full length

<400>  55

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Met
        35                  40                  45

Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Phe Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125
```

```
Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380
```

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 56
<211> 120
<212> PRT
<213> artificial

<220>
<223> hz208F2 heavy chain H037, VH

<400> 56

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

Gly Trp Ile Trp Pro Gly Asp Gly Ser Thr Lys Tyr Ala Gln Lys Phe
50 55 60

Gln Gly Arg Val Thr Met Thr Arg Asp Lys Ser Ser Ser Thr Val Tyr
65 70 75 80

Met Glu Leu Ser Ser Leu Arg Ser Glu Asp Thr Ala Val Tyr Phe Cys
85 90 95

Ala Ser Pro Met Ile Thr Pro Asn Tyr Ala Met Asp Tyr Trp Gly Gln
100 105 110

Gly Thr Leu Val Thr Val Ser Ser
115 120

<210> 57
<211> 107
<212> PRT
<213> artificial

<220>
<223> hz208F2 light chain L018, VL

<400> 57

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Lys Tyr
20 25 30

Leu Asn Trp Tyr Gln Gln Lys Pro Gly Lys Ala Pro Lys Leu Leu Ile
35 40 45

Tyr Tyr Thr Ser Arg Leu Gln Ser Gly Val Pro Ser Arg Phe Ser Gly
50 55 60

Arg Gly Ser Gly Thr Asp Tyr Ser Leu Thr Ile Ser Ser Leu Gln Pro
65 70 75 80

Glu Asp Phe Ala Thr Tyr Phe Cys Gln Gln Gly Ser Thr Leu Pro Tyr
85 90 95

Thr Phe Gly Gly Gly Thr Lys Val Glu Ile Lys
100 105

<210> 58
<211> 449
<212> PRT
<213> artificial

<220>
<223> hz208F2 heavy chain H037 full length

<400> 58

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

Gly Trp Ile Trp Pro Gly Asp Gly Ser Thr Lys Tyr Ala Gln Lys Phe
50 55 60

```
Gln Gly Arg Val Thr Met Thr Arg Asp Lys Ser Ser Ser Thr Val Tyr
65                  70                  75                  80

Met Glu Leu Ser Ser Leu Arg Ser Glu Asp Thr Ala Val Tyr Phe Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
```

```
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly



<210>  59
<211>  214
<212>  PRT
<213>  artificial

<220>
<223>  hz208F2 light chain L018 full length

<400>  59

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Lys Tyr
            20                  25                  30

Leu Asn Trp Tyr Gln Gln Lys Pro Gly Lys Ala Pro Lys Leu Leu Ile
        35                  40                  45

Tyr Tyr Thr Ser Arg Leu Gln Ser Gly Val Pro Ser Arg Phe Ser Gly
    50                  55                  60
```

```
Arg Gly Ser Gly Thr Asp Tyr Ser Leu Thr Ile Ser Ser Leu Gln Pro
65                  70                  75                  80

Glu Asp Phe Ala Thr Tyr Phe Cys Gln Gln Gly Ser Thr Leu Pro Tyr
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  60
<211>  107
<212>  PRT
<213>  artificial

<220>
<223>  hz208F2 light chain L021, VL

<400>  60

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Lys Tyr
            20                  25                  30

Leu Asn Trp Tyr Gln Gln Lys Pro Gly Lys Ala Pro Lys Leu Leu Ile
        35                  40                  45
```

Tyr Tyr Thr Ser Arg Leu His Ser Gly Val Pro Ser Arg Phe Ser Gly
50 55 60

Arg Gly Ser Gly Thr Asp Phe Ser Leu Thr Ile Ser Ser Leu Gln Pro
65 70 75 80

Glu Asp Phe Ala Thr Tyr Phe Cys Gln Gln Gly Ser Thr Leu Pro Tyr
85 90 95

Thr Phe Gly Gly Gly Thr Lys Val Glu Ile Lys
100 105

<210> 61
<211> 214
<212> PRT
<213> artificial

<220>
<223> hz208F2 light chain L021 full length

<400> 61

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Lys Tyr
20 25 30

Leu Asn Trp Tyr Gln Gln Lys Pro Gly Lys Ala Pro Lys Leu Leu Ile
35 40 45

Tyr Tyr Thr Ser Arg Leu His Ser Gly Val Pro Ser Arg Phe Ser Gly
50 55 60

Arg Gly Ser Gly Thr Asp Phe Ser Leu Thr Ile Ser Ser Leu Gln Pro
65 70 75 80

Glu Asp Phe Ala Thr Tyr Phe Cys Gln Gln Gly Ser Thr Leu Pro Tyr
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

```
Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  62
<211>  120
<212>  PRT
<213>  artificial

<220>
<223>  hz208F2 heavy chain H047, VH

<400>  62

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Met
        35                  40                  45

Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Ser Asn Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser
        115                 120


<210>  63
```

<211> 449
<212> PRT
<213> artificial

<220>
<223> hz208F2 heavy chain H047 full length

<400> 63

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Met
        35                  40                  45

Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Ser Asn Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220
```

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210> 64

<211> 120
<212> PRT
<213> artificial

<220>
<223> hz208F2 heavy chain H049, VH

<400> 64

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Trp Ile Trp Pro Gly Asp Gly Ser Thr Lys Tyr Ala Gln Lys Phe
    50                  55                  60

Gln Gly Arg Val Thr Met Thr Arg Asp Lys Ser Ser Ser Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser
        115                 120
```

<210> 65
<211> 449
<212> PRT
<213> artificial

<220>
<223> hz208F2 heavy chain H049 full length

<400> 65

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45
```

```
Gly Trp Ile Trp Pro Gly Asp Gly Ser Thr Lys Tyr Ala Gln Lys Phe
    50                  55                  60

Gln Gly Arg Val Thr Met Thr Arg Asp Lys Ser Ser Ser Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300
```

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 66
<211> 120
<212> PRT
<213> artificial

<220>
<223> hz208F2 heavy chain H051, VH

<400> 66

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Ser Asn Thr Val Tyr
65                  70                  75                  80

Met Glu Leu Ser Ser Leu Arg Ser Glu Asp Thr Ala Val Tyr Phe Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser
        115                 120

<210>  67
<211>  449
<212>  PRT
<213>  artificial

<220>
<223>  hz208F2 heavy chain H051 full length

<400>  67

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Ser Asn Thr Val Tyr
65                  70                  75                  80

Met Glu Leu Ser Ser Leu Arg Ser Glu Asp Thr Ala Val Tyr Phe Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala

```
        130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380
```

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 68
<211> 120
<212> PRT
<213> artificial

<220>
<223> hz208F2 heavy chain H052, VH

<400> 68

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

Gly Trp Ile Trp Pro Gly Asp Gly Ser Thr Lys Tyr Ala Gln Lys Phe
50 55 60

Gln Gly Arg Val Thr Met Thr Arg Asp Thr Ser Thr Ser Thr Val Tyr
65 70 75 80

Met Glu Leu Ser Ser Leu Arg Ser Glu Asp Thr Ala Val Tyr Phe Cys
85 90 95

Ala Ser Pro Met Ile Thr Pro Asn Tyr Ala Met Asp Tyr Trp Gly Gln
100 105 110

Gly Thr Leu Val Thr Val Ser Ser
115 120

<210> 69

<211> 449
<212> PRT
<213> artificial

<220>
<223> hz208F2 heavy chain H052 full length

<400> 69

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

Gly Trp Ile Trp Pro Gly Asp Gly Ser Thr Lys Tyr Ala Gln Lys Phe
50 55 60

Gln Gly Arg Val Thr Met Thr Arg Asp Thr Ser Thr Ser Thr Val Tyr
65 70 75 80

Met Glu Leu Ser Ser Leu Arg Ser Glu Asp Thr Ala Val Tyr Phe Cys
85 90 95

Ala Ser Pro Met Ile Thr Pro Asn Tyr Ala Met Asp Tyr Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Arg Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 70

<211> 120
<212> PRT
<213> artificial

<220>
<223> hz208F2 heavy chain H057, VH

<400> 70

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Met
        35                  40                  45

Gly Trp Ile Trp Pro Gly Asp Gly Ser Thr Lys Tyr Ala Gln Lys Phe
    50                  55                  60

Gln Gly Arg Val Thr Met Thr Arg Asp Lys Ser Thr Asn Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser
        115                 120
```

<210> 71
<211> 449
<212> PRT
<213> artificial

<220>
<223> hz208F2 heavy chain H057 full length

<400> 71

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Met
        35                  40                  45
```

Gly Trp Ile Trp Pro Gly Asp Gly Ser Thr Lys Tyr Ala Gln Lys Phe
50 55 60

Gln Gly Arg Val Thr Met Thr Arg Asp Lys Ser Thr Asn Thr Val Tyr
65 70 75 80

Met Glu Leu Ser Ser Leu Arg Ser Glu Asp Thr Ala Val Tyr Tyr Cys
85 90 95

Ala Ser Pro Met Ile Thr Pro Asn Tyr Ala Met Asp Tyr Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Arg Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 72
<211> 120
<212> PRT
<213> artificial

<220>
<223> hz208F2 heavy chain H068, VH

<400> 72

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

```
Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Thr Asn Thr Val Tyr
65                  70                  75                  80

Met Glu Leu Ser Ser Leu Arg Ser Glu Asp Thr Ala Val Tyr Phe Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser
        115                 120


<210>  73
<211>  449
<212>  PRT
<213>  artificial

<220>
<223>  hz208F2 heavy chain H068 full length

<400>  73

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Thr Asn Thr Val Tyr
65                  70                  75                  80

Met Glu Leu Ser Ser Leu Arg Ser Glu Asp Thr Ala Val Tyr Phe Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
```

```
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380
```

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 74
<211> 120
<212> PRT
<213> artificial

<220>
<223> hz208F2 heavy chain H070, VH

<400> 74

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Met His Trp Val Arg Gln Ala Pro Gly Gln Gly Leu Glu Trp Met
35 40 45

Gly Trp Ile Trp Pro Gly Asp Gly Ser Thr Lys Tyr Ala Gln Lys Phe
50 55 60

Gln Gly Arg Val Thr Met Thr Arg Asp Thr Ser Thr Ser Thr Val Tyr
65 70 75 80

Met Glu Leu Ser Ser Leu Arg Ser Glu Asp Thr Ala Val Tyr Tyr Cys
85 90 95

Ala Ser Pro Met Ile Thr Pro Asn Tyr Ala Met Asp Tyr Trp Gly Gln
100 105 110

Gly Thr Leu Val Thr Val Ser Ser
115 120

<210> 75

<211> 449
<212> PRT
<213> artificial

<220>
<223> hz208F2 heavy chain H070 full length

<400> 75

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220
```

```
Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly
```

<210>  76

<211> 120
<212> PRT
<213> artificial

<220>
<223> hz208F2 heavy chain H071, VH

<400> 76

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Thr Asn Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser
        115                 120
```

<210> 77
<211> 449
<212> PRT
<213> artificial

<220>
<223> hz208F2 heavy chain H071 full length

<400> 77

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45
```

```
Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Thr Asn Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300
```

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly

<210> 78
<211> 120
<212> PRT
<213> artificial

<220>
<223> hz208F2 heavy chain H076, VH

<400> 78

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Tyr Ile His Trp Val Arg Gln Ala Pro Gly Gln Gly Leu Glu Trp Met
35 40 45

```
Gly Trp Ile Trp Pro Gly Asp Gly Ser Thr Lys Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Ser Pro Met Ile Thr Pro Asn Tyr Ala Met Asp Tyr Trp Gly Gln
            100                 105                 110

Gly Thr Leu Val Thr Val Ser Ser
        115                 120
```

## Description

La présente invention porte sur l'utilisation d'un ADC anti-IGF1R (ADC-A) pour le traitement de maladies prolifératives, telles que le cancer, caractérisée en ce que ADC-A est administré de façon concomitante ou postérieurement au docétaxel.

Le traitement maladies hyperprolifératives, telles que le cancer, constitue un réel challenge de santé public. Il s'agit, bien entendu, de trouver en premier lieu des médicaments anti-tumoraux efficaces. Mais il s'agit aussi de prévoir des solutions à long terme en cas d'apparition de résistance, phénomène fréquent, à ces premiers traitements.

Le docétaxel est un diterpène utilisé comme médicament anti-cancéreux qui agit sur les microtubules des cellules cancéreuses et entraine un blocage de la mitose. L'efficacité du docétaxel a été prouvé dans un assez grand nombre de tumeurs notamment cancer du sein, cancer du poumon, cancer de la prostate. Cependant un grand nombre de patients développe au fil du temps une résistance au docétaxel aboutissant à l'échec du traitement.

Le récepteur du facteur de croissance 1 analogue à l'insuline appelé IGF-1R (ou IGF1R) est un récepteur à activité tyrosine kinase ayant une homologie de 70 % avec le récepteur à l'insuline IR. IGF-IR est une glycoprotéine de poids moléculaire d'environ 350 000. Il s'agit d'un récepteur hétérotétramère dont chaque moitié - liée par des ponts disulfure - est composée d'une sous-unité extracellulaire $\alpha$ et d'une sous-unité transmembranaire $\beta$. IGF-IR lie IGF1 et IGF2 avec une affinité très élevée (Kd #1 nM) et est capable de se lier à l'insuline avec une affinité 100 à 1000 fois inférieure.

Des protéines tyrosine kinase cytoplasmiques sont activées par la liaison du ligand au domaine extracellulaire d'IGF-1R. L'activation de ces kinases entraine à son tour l'activation de différents substrats intracellulaires, dont IRS-1, IRS-2, She et Grb 10. Les protéines IRS interviennent, par l'activation de nombreux effecteurs en aval, dans de nombreuses voies induisant la croissance et la différenciation cellulaires. Il semble, en outre, que la voie principalement impliquée dans la protection contre l'apoptose soit la voie des phosphatidyl-inositol 3-kinases (PI3-kinases).

Le rôle du système IGF dans la cancérogenèse a fait l'objet de recherches intensives au cours des dix dernières années. En effet, il est bien établi qu'une activation de l'IGF-IR conduit, dans une grande variété de cellules, à une croissance anormale des cellules indépendante et à la formation de tumeurs. IGF-IR est ainsi exprimé dans une grande variété de tumeurs et de lignées tumorales.

Afin de traiter ce type de cancer, des thérapies ciblées visant IGF1R ont été mises au point. Il a ainsi été prouvé que l'utilisation d'ADC (antibody-drug conjugate) ciblant IGF-1R permet de réduire, voire d'abolir la croissance tumorale. De tels ADC ont notamment été décrits dans la demande de brevet WO2015162291 en particulier l'ADC décrit ci-après et nommé ADC-A.

Il existe un réel besoin de traitement efficace des maladies hyper-prolifératives. Il est par ailleurs nécessaire d'identifier des combinaisons de thérapies permettant d'échapper au phénomène d'apparition de résistance au sein des tumeurs.

De façon surprenante, la présente invention a permis de montrer que l'utilisation d'un ADC anti-IGF1R en combinaison avec le docétaxel permet d'obtenir un effet synergique dans le traitement des tumeurs. De façon toute aussi surprenante il a été montré que l'utilisation d'un ADC anti-IGF1R permet de traiter les tumeurs devenues résistantes après un traitement au docétaxel.

**Légende de la Figure** :

**FIG 1:** Evolution du volume tumoral moyen en $mm^3$ en fonction du temps en jours chez des souris porteuses d'une tumeur MCF-7 d'environ 150 $mm^3$ après A. Traitement par du docétaxel à 9mg/kg une fois toutes les deux semaines pendant 5 cycles (les trois courbes confondues correspondent à trois séries de 5 souris traitées de façon identique et au même moment) et B. Traitement des souris rendues résistantes au docétaxel au jour 70 par : groupe 1, du docétaxel 9 mg/kg toutes les deux semaines (courbe points ronds) et ; groupe 2, une injection de ADC-A à 3 mg/kg (courbe points triangles).

La présente invention a ainsi pour objet un ADC anti-IGF1R (ADC-A) pour son utilisation dans le traitement de maladies prolifératives, telles que le cancer, caractérisé en ce que ADC-A est administré de façon concomitante ou postérieurement à du docétaxel.

La présente invention a également pour objet l'utilisation d'un ADC anti-IGF1R (ADC-A) pour le traitement de maladies prolifératives, telles que le cancer, caractérisée en ce que ADC-A est administré de façon concomitante ou postérieurement à du docétaxel.

La présente invention a également pour objet l'utilisation d'un ADC anti-IGF1R (ADC-A) pour la préparation d'un médicament destiné au traitement de maladies prolifératives, telles que le cancer, caractérisée en ce que ADC-A est administré de façon concomitante ou postérieurement à du docétaxel.

La présente invention a également pour objet une méthode de traitement d'une maladie proliférative, telle que le cancer, comprenant l'administration à un patient en ayant besoin d'une quantité efficace d'un ADC anti-IGF1R (ADC-A) et de docétaxel, caractérisée en ce que ADC-A est administré de façon concomitante ou postérieurement au docétaxel.

Docétaxel

Par docétaxel, selon l'invention, on entend le composé de formule suivante :

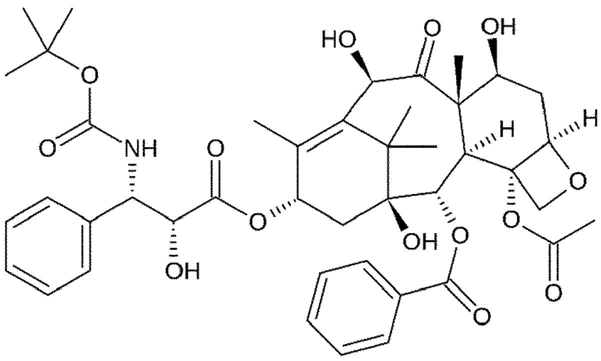

ainsi que ses sels et solvates pharmaceutiquement acceptables, notamment son trihydrate.

Le docétaxel est notamment vendu sous le nom de marque Taxotere®.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sel ou solvate pharmaceutiquement acceptable » d'un composé, un sel ou solvate qui est pharmaceutiquement acceptable, comme défini ici, et qui possède l'activité pharmacologique souhaitée du composé parent.

Les sels pharmaceutiquement acceptables comprennent notamment :

(1) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide formique, l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, et

(2) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique pharmaceutiquement acceptable telle que la diéthanolamine, l'éthanolamine, N-méthylglucamine, la triéthanolamine, la trométhamine et similaires ; ou avec une base inorganique pharmaceutiquement acceptable telle que l'hydroxyde d'aluminium, l'hydroxyde de calcium, l'hydroxyde de potassium, le carbonate de sodium, l'hydroxyde de sodium et similaires.

Des solvates acceptables pour l'utilisation pharmaceutique des composés selon la présente invention incluent des solvates conventionnels tels que ceux formés, durant la dernière étape du procédé de préparation des composés selon l'invention, avec le(s) solvant(s) de réaction. A titre d'exemple, il peut être fait mention des solvates formés avec l'eau (appelés communément hydrates) ou avec l'éthanol. Il peut s'agir par un exemple d'un trihydrate.

ADC-A

ADC-A est un ADC anti-IGF1R de formule suivante

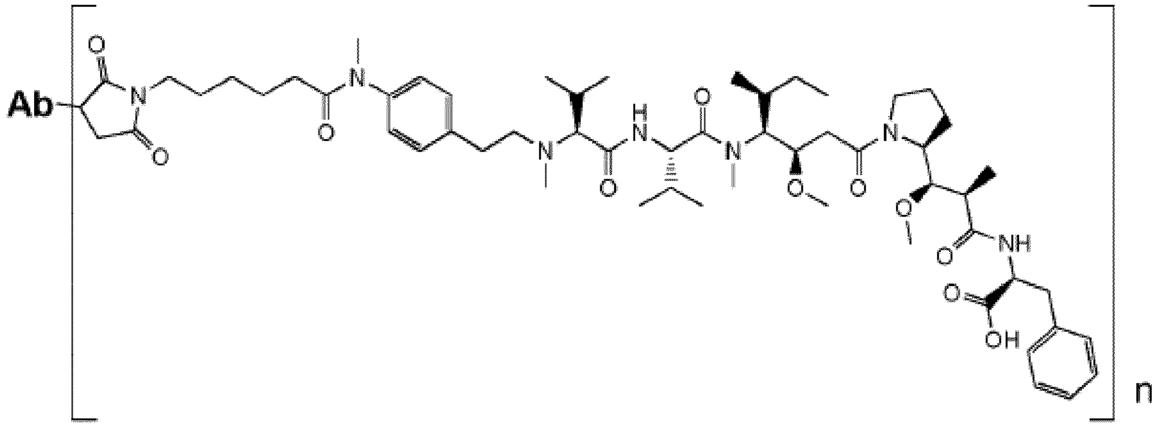

dans laquelle Ab est un anticorps anti-IGF1R comprenant les trois CDR (régions complémentaires déterminantes - complementarity determining régions en anglais) de chaînes lourdes de séquences SEQ ID No 1, 2 et 3 et les trois CDR de chaîne légère de séquences SEQ ID No 4, 5 et 6,et n est compris entre 1 et 12, notamment est égale à 2 ou 4.

Les termes « anticorps », « ab », « Ab », « Mab » ou « immunoglobuline » sont utilisés indifféremment dans le sens le plus large et comprennent les anticorps monoclonaux, les anticorps isolés, modifiés ou recombinants (par exemple, les anticorps monoclonaux complets ou intacts), les anticorps polyclonaux, les anticorps multivalents ou des anticorps multispécifiques (par exemple des anticorps bispécifiques) ainsi que les fragments d'anticorps de ceux-ci, pour autant qu'ils présentent l'activité biologique souhaitée, en particulier les fragments de ceux-ci se liant à l'antigène. Il s'agit plus particulièrement d'anticorps monoclonaux ou éventuellement de fragments de ceux-ci se liant à l'antigène.

Le terme « fragment se liant à l'antigène » d'un anticorps selon l'invention est destiné à indiquer tout peptide, polypeptide ou protéine conservant l'aptitude à se lier à la cible (également appelée généralement antigène) de l'anticorps.

Tel qu'utilisé dans la présente description, l'expression « anticorps anti-IGF-1R » désigne un anticorps capable de se lier à IGF-1R, plus particulièrement à un épitope localisé dans IGF-1R, de préférence le domaine extracellulaire de l'IGF-1R, plus préférablement l'IGF-1R humain (SEQ ID No 50) et encore plus préférablement le domaine extracellulaire de l'IGF-1R humain (SEQ ID No 51), et encore plus préférablement la partie N-terminale du domaine extracellulaire de l'IGF-1R humain (SEQ ID No 52), ou l'une quelconque de ses séquences variantes naturelles.

Selon un mode de réalisation particulier de l'invention, l'anticorps selon l'invention comprend les trois CDR de chaînes lourdes comprenant ou consistant en les séquences SEQ ID No 1, 2 et 3, ou toute séquence présentant au moins 80%, de préférence 85%, 90%, 95% et 98% d'identité avec SEQ ID No 1, 2 ou 3; et les trois CDR de chaînes légères comprenant ou consistant en les séquences SEQ ID No 4, 5 et 6, ou toute séquence présentant au moins 80%, de préférence 85%, 90%, 95% et 98% d'identité avec la SEQ ID No 4, 5 ou 6.

Les pourcentages d'identité auxquels il est fait référence dans le cadre de l'exposé de la présente invention sont déterminés sur la base d'un alignement global des séquences à comparer, c'est-à-dire sur un alignement des séquences prises dans leur intégralité sur toute la longueur en utilisant tout algorithme bien connu de l'homme du métier tel que l'algorithme de Needleman et Wunsch-1970. Cette comparaison de séquences peut être effectuée à l'aide de tout logiciel bien connu de l'homme du métier : par exemple le logiciel needle en utilisant le paramètre « Gap open » égal à 10,0, le paramètre « Gap extend » égal à 0,5 et une matrice « Blosum 62 ». Le logiciel needle est par exemple disponible sur le site internet ebi.ac.uk world wide sous la dénomination « Align ».

Lorsque l'anticorps selon l'invention possède une séquence d'acides aminés qui n'est pas 100% identique à la séquence de référence mais qui possède au moins 80%, de préférence 85%, 90%, 95% et 98% d'identité avec une telle séquence de référence, elle peut présenter des insertions, délétions ou substitutions par rapport à la séquence de référence. Lorsqu'il s'agit de substitutions, la substitution est de préférence réalisée par un acide aminé « équivalent », c'est-à-dire tout acide aminé dont la structure est proche de celle de l'acide aminé d'origine et est donc peu probable de modifier les activités biologiques de l'anticorps. Des exemples de telles substitutions sont présentés dans le tableau suivant :

| Acide aminé d'origine | Substitution(s) |
|---|---|
| Ala (A) | Val, Gly, Pro |
| Arg (R) | Lys, His |
| Asn (N) | Gln |

(suite)

| Acide aminé d'origine | Substitution(s) |
|---|---|
| Asp (D) | Glu |
| Cys (C) | Ser |
| Gln (Q) | Asn |
| Glu (G) | Asp |
| Gly (G) | Ala |
| His (H) | Arg |
| Ile (I) | Leu |
| Leu (L) | Ile, Val, Met |
| Lys (K) | Arg |
| Met (M) | Leu |
| Phe (F) | Tyr |
| Pro (P) | Ala |
| Ser (S) | Thr, Cys |
| Thr (T) | Ser |
| Trp (W) | Tyr |
| Tyr (Y) | Phe, Trp |
| Val (V) | Leu, Ala |

Le tableau 1 ci-dessous illustre les séquences des CDR pour les anticorps préférés :

Tableau 1

| | **Chaine lourde** | **Chaine légère** | **SEQ ID No** |
|---|---|---|---|
| Consensus | CDR-H1 | | 1 |
| | CDR-H2 | | 2 |
| | CDR-H3 | | 3 |
| | | CDR-L1 | 4 |
| | | CDR-L2 | 5 |
| | | CDR-L3 | 6 |
| 208F2 | CDR-H1 | | 7 |
| | CDR-H2 | | 2 |
| | CDR-H3 | | 3 |
| | | CDR-L1 | 9 |
| | | CDR-L2 | 5 |
| | | CDR-L3 | 11 |

(suite)

| | Chaine lourde | Chaine légère | SEQ ID No |
|---|---|---|---|
| 212A11 | CDR-H1 | | 7 |
| | CDR-H2 | | 2 |
| | CDR-H3 | | 3 |
| | | CDR-L1 | 10 |
| | | CDR-L2 | 5 |
| | | CDR-L3 | 11 |
| 214F8 & 213B10 | CDR-H1 | | 7 |
| | CDR-H2 | | 2 |
| | CDR-H3 | | 3 |
| | | CDR-L1 | 9 |
| | | CDR-L2 | 5 |
| | | CDR-L3 | 12 |
| 219D6 | CDR-H1 | | 8 |
| | CDR-H2 | | 2 |
| | CDR-H3 | | 3 |
| | | CDR-L1 | 9 |
| | | CDR-L2 | 5 |
| | | CDR-L3 | 11 |

Selon un aspect spécifique, l'anticorps est un anticorps murin caractérisé en ce qu'il comprend également des régions constantes de chaîne légère et de chaîne lourde dérivées d'un anticorps d'une espèce hétérologue chez la souris, notamment l'homme.

Selon un aspect spécifique de l'invention, l'anticorps est un anticorps chimérique (c) caractérisé en ce qu'il comprend également des régions constantes de chaînes légères et lourdes dérivées d'un anticorps d'une espèce hétérologue avec la souris, notamment humaine.

Un anticorps chimérique est un anticorps contenant une région variable naturelle (chaîne légère et chaîne lourde) dérivée d'un anticorps d'une espèce donnée en combinaison avec des régions constantes de la chaîne légère et la chaîne lourde d'un anticorps d'une espèce hétérologue à ladite espèce donnée.

Selon un mode de réalisation de l'invention, l'anticorps est choisi parmi :

a) un anticorps comprenant les trois CDR à chaînes lourdes de séquences SEQ ID No 7, 2 et 3 et les trois CDR à chaînes légères de séquences SEQ ID No 9, 5 et 11 ;

b) un anticorps comprenant les trois CDR à chaînes lourdes de séquences SEQ ID No 7, 2 et 3 et les trois CDR à chaînes légères de séquences SEQ ID No 10, 5 et 11 ;

c) un anticorps comprenant les trois CDR à chaînes lourdes de séquences SEQ ID No 7, 2 et 3 et les trois CDR à chaînes légères de séquences SEQ ID No 9, 5 et 12 ; et

d) un anticorps comprenant les trois CDR à chaînes lourdes de séquences SEQ ID No 8, 2 et 3 et les trois CDR à chaînes légères de séquences SEQ ID No 9, 5 et 11.

Dans un mode de réalisation préféré mais non limitatif, l'anticorps est choisi parmi :

a) un anticorps comprenant un domaine variable de chaîne lourde de séquence SEQ ID No 13 ou toute séquence présentant au moins 80% d'identité avec la SEQ ID No 13 et les trois CDR de chaîne légère de séquences SEQ

ID No 9, 5 et 11 ;

b) un anticorps comprenant un domaine variable de chaîne lourde de séquence SEQ ID No 14 ou toute séquence présentant au moins 80% d'identité avec la SEQ ID No 14 et les trois CDR de chaîne légère de séquences SEQ ID No 10, 5 et 11 ;

c) un anticorps comprenant un domaine variable de chaîne lourde de séquence SEQ ID No 15 ou toute séquence présentant au moins 80% d'identité avec la SEQ ID No 15 et les trois CDR de chaîne légère de séquences SEQ ID No 9, 5 et 12 ;

d) un anticorps comprenant un domaine variable de chaîne lourde de séquence SEQ ID No 16 ou toute séquence présentant au moins 80% d'identité avec la SEQ ID No 16 et les trois CDR de chaîne légère de séquences SEQ ID No 9, 5 et 11 ; et

e) un anticorps comprenant un domaine variable de chaîne lourde de séquence SEQ ID No 17 ou toute séquence présentant au moins 80% d'identité avec la SEQ ID No 17 et les trois CDR de chaîne légère de séquences SEQ ID No 9, 5 et 12.

Par « toute séquence présentant au moins 80%, de préférence 85%, 90%, 95% et 98% d'identité avec SEQ ID No 13 à 17 », on entend respectivement les séquences présentant les trois CDR à chaînes lourdes SEQ ID No 1, 2 et 3 et, en outre, présentant au moins 80%, de préférence 85%, 90%, 95% et 98%, d'identité avec la séquence complète SEQ ID No 13 à 17 en dehors des séquences correspondant aux CDR (c'est-à-dire SEQ ID No 1, 2 et 3).

Selon un mode de réalisation de l'invention, l'anticorps est choisi parmi :

a) un anticorps comprenant un domaine variable de chaîne lourde de séquence SEQ ID No 13 et les trois CDR de chaîne légère de séquence SEQ ID No 9, 5 et 11 ;

b) un anticorps comprenant un domaine variable de chaîne lourde de séquence SEQ ID No 14 et les trois CDR de chaîne légère de séquence SEQ ID No 10, 5 et 11 ;

c) un anticorps comprenant un domaine variable de chaîne lourde de séquence SEQ ID No 15 et les trois CDR de chaîne légère de séquence SEQ ID No 9, 5 et 12 ;

d) un anticorps comprenant un domaine variable de chaîne lourde de séquence SEQ ID No 16 et les trois CDR de chaîne légère de séquence SEQ ID No 9, 5 et 11 ; et

e) un anticorps comprenant un domaine variable de chaîne lourde de séquence SEQ ID No 17 et les trois CDR de chaîne légère de séquence SEQ ID No 9, 5 et 12.

Dans un autre mode de réalisation préféré mais non limitatif, l'anticorps est choisi parmi :

a) un anticorps comprenant un domaine variable de chaîne légère de séquence SEQ ID No 18 ou toute séquence présentant au moins 80% d'identité avec la SEQ ID No 18 et les trois CDR de chaînes lourdes de séquences SEQ ID No 7, 2 et 3 ;

b) un anticorps comprenant un domaine variable de chaîne légère de séquence SEQ ID No 19 ou toute séquence présentant au moins 80% d'identité avec la SEQ ID No 19 et les trois CDR de chaînes lourdes de séquences SEQ ID No 7, 2 et 3 ;

c) un anticorps comprenant un domaine variable de chaîne légère de séquence SEQ ID No 20 ou toute séquence présentant au moins 80% d'identité avec la SEQ ID No 20 et les trois CDR de chaîne lourde de séquences SEQ ID No 7, 2 et 3 ;

d) un anticorps comprenant un domaine variable de chaîne légère de séquence SEQ ID No 21 ou toute séquence présentant au moins 80% d'identité avec la SEQ ID No 21 et les trois CDR de chaînes lourdes de séquences SEQ ID No 8, 2 et 3 ; et

e) un anticorps comprenant un domaine variable de chaîne légère de séquence SEQ ID No 22 ou toute séquence

présentant au moins 80% d'identité avec la SEQ ID No 22 et les trois CDR de chaîne lourde des séquences SEQ ID No 7, 2 et 3.

Par « toute séquence présentant au moins 80%, de préférence 85%, 90%, 95% et 98% d'identité avec la SEQ ID No 18 à 22 », on entend respectivement les séquences présentant les trois CDR à chaînes légères SEQ ID No 4, 5 et 6 et, en outre, présentant au moins 80%, de préférence 85%, 90%, 95% et 98%, d'identité avec la séquence complète SEQ ID No 18 à 22 en dehors des séquences correspondant aux CDR (c'est-à-dire SEQ ID No 4, 5 et 6).

Selon un mode de réalisation de l'invention, l'anticorps est choisi parmi :

a) un anticorps comprenant un domaine variable de chaîne légère de séquence SEQ ID No 18 et les trois CDR de chaîne lourde de séquence SEQ ID No 7, 2 et 3 ;

b) un anticorps comprenant un domaine variable de chaîne légère de séquence SEQ ID No 19 et les trois CDR de chaîne lourde de séquence SEQ ID No 7, 2 et 3 ;

c) un anticorps comprenant un domaine variable de chaîne légère de séquence SEQ ID No 20 et les trois CDR de chaîne lourde de séquence SEQ ID No 7, 2 et 3 ;

d) un anticorps comprenant un domaine variable de chaîne légère de séquence SEQ ID No 21 et les trois CDR de chaîne lourde de séquence SEQ ID No 8, 2 et 3 ; et

e) un anticorps comprenant un domaine variable de chaîne légère de séquence SEQ ID No 22 et les trois CDR de chaîne lourde de séquence SEQ ID No 7, 2 et 3.

Selon un mode de réalisation de l'invention, l'anticorps est un anticorps choisi parmi

a) un anticorps comprenant un domaine variable de chaîne lourde de séquence SEQ ID No 13 ou toute séquence présentant au moins 80% d'identité avec la SEQ ID No 13 et un domaine variable de chaîne légère de séquence SEQ ID No 18 ou toute séquence présentant au moins 80% d'identité avec SEQ ID No 18 ;

b) un anticorps comprenant un domaine variable de chaîne lourde de séquence SEQ ID No 14 ou toute séquence présentant au moins 80% d'identité avec la SEQ ID No 14 et un domaine variable de chaîne légère de séquence SEQ ID No 19 ou toute séquence présentant au moins 80% d'identité avec SEQ ID No 19 ;

c) un anticorps comprenant un domaine variable de chaîne lourde de séquence SEQ ID No 15 ou toute séquence présentant au moins 80% d'identité avec la SEQ ID No 15 et un domaine variable de chaîne légère de séquence SEQ ID No 20 ou toute séquence présentant au moins 80% d'identité avec SEQ ID No 20 ;

d) un anticorps comprenant un domaine variable de chaîne lourde de séquence SEQ ID No 16 ou toute séquence présentant au moins 80% d'identité avec la SEQ ID No 16 et un domaine variable de chaîne légère de séquence SEQ ID No 21 ou toute séquence présentant au moins 80% d'identité avec SEQ ID No 21 ; et

e) un anticorps comprenant un domaine variable de chaîne lourde de séquence SEQ ID No 17 ou toute séquence présentant au moins 80% d'identité avec la SEQ ID No 17 et un domaine variable de chaîne légère de séquence SEQ ID No 22 ou toute séquence présentant au moins 80% d'identité avec SEQ ID No 22.

Les anticorps chimériques décrits ici peuvent également être caractérisés par le domaine constant et, plus particulièrement, lesdits anticorps chimériques peuvent être sélectionnés ou conçus tels que, sans limitation, IgG1, IgG2, IgG3, IgM, IgA, IgD ou IgE. Plus préférentiellement, dans le cadre de la présente invention, lesdits anticorps chimériques sont IgG1 ou IgG4.

Selon un mode de réalisation de l'invention, l'anticorps est un anticorps chimérique comprenant les domaines variables de chaîne lourde (VH) et de chaîne légère (VL) tels que décrits ci-dessus au format IgG1. Plus préférentiellement, ledit anticorps chimérique comprend un domaine constant pour le VH de séquence SEQ ID No 43 et un domaine Kappa pour le VL de séquence SEQ ID No 45.

Selon un mode de réalisation de l'invention, l'anticorps est un anticorps chimérique comprenant les domaines variables VH et VL tels que décrits ci-dessus au format IgG4. Plus préférentiellement, ledit anticorps chimérique comprend un domaine constant pour le VH de séquence SEQ ID No 44 et un domaine Kappa pour le VL de séquence SEQ ID No 45.

Dans un autre mode de réalisation préféré mais non limitatif, l'anticorps est choisi parmi :

a) un anticorps comprenant ou consistant en une chaîne lourde de séquence SEQ ID No 23 ou toute séquence présentant au moins 80% d'identité avec la SEQ ID No 23 et une chaîne légère de séquence SEQ ID No 28 ou toute séquence présentant au moins 80% d'identité avec SEQ ID No 28 ;

b) un anticorps comprenant ou consistant en une chaîne lourde de séquence SEQ ID No 24 ou toute séquence présentant au moins 80% d'identité avec la SEQ ID No 24 et une chaîne légère de séquence SEQ ID No 29 ou toute séquence présentant au moins 80% d'identité avec SEQ ID No 29 ;

c) un anticorps comprenant ou consistant en une chaîne lourde de séquence SEQ ID No 25 ou toute séquence présentant au moins 80% d'identité avec la SEQ ID v 25 et une chaîne légère de séquence SEQ ID v 30 ou toute séquence présentant au moins 80% d'identité avec SEQ ID No 30 ;

d) un anticorps comprenant ou consistant en une chaîne lourde de séquence SEQ ID No 26 ou toute séquence présentant au moins 80% d'identité avec la SEQ ID No 26 et une chaîne légère de séquence SEQ ID No 31 ou toute séquence présentant au moins 80% % d'identité avec SEQ ID No 31 ; et

e) un anticorps comprenant ou consistant en une chaîne lourde de séquence SEQ ID No 27 ou toute séquence présentant au moins 80% d'identité avec la SEQ ID No 27 et une chaîne légère de séquence SEQ ID No 32 ou toute séquence présentant au moins 80% d'identité avec SEQ ID No 32.

Pour plus de clarté, le tableau 2 suivant illustre les séquences de VH et VL, respectivement, pour les anticorps chimériques préférés.

Tableau 2

| | **Chaine lourde** | **Chaine légère** | **SEQ ID No.** |
|---|---|---|---|
| c208F2 | Domaine variable (VH) | | 13 |
| | | Domaine variable (VL) | 18 |
| | Longueur complète | | 23 |
| | | Longueur complète | 28 |
| c212A11 | Domaine variable (VH) | | 14 |
| | | Domaine variable (VL) | 19 |
| | Longueur complète | | 24 |
| | | Longueur complète | 29 |
| c214F8 | Domaine variable (VH) | | 15 |
| | | Domaine variable (VL) | 20 |
| | Longueur complète | | 25 |
| | | Longueur complète | 30 |
| c219D6 | Domaine variable (VH) | | 16 |
| | | Domaine variable (VL) | 21 |
| | Longueur complète | | 26 |
| | | Longueur complète | 31 |
| c213B10 | Domaine variable (VH) | | 17 |
| | | Domaine variable (VL) | 22 |
| | Longueur complète | | 27 |
| | | Longueur complète | 32 |

Selon un autre aspect spécifique de la présente invention, l'anticorps est un anticorps humanisé caractérisé en ce que les régions constantes de la chaîne légère et de la chaîne lourde dérivées de l'anticorps humain sont respectivement la région lambda ou kappa et les régions gamma-1, gamma-2 ou gamma-4.

Dans un mode de réalisation préféré, l'anticorps comprend un domaine variable de chaîne lourde (VH) ayant :

i) les CDR-H1, CDR-H2 et CDR-H3 des séquences SEQ ID No 7, 2 et 3, respectivement, et

ii) les FR1, FR2 et FR3 dérivés de la lignée germinale humaine IGHV1-46*01 (SEQ ID No 46), et

iii) le FR4 dérivé de la lignée germinale humaine IGHJ4*01 (SEQ ID No 48).

Dans un mode de réalisation préféré, l'anticorps comprend un domaine variable de chaîne légère (VL) ayant :

i) les CDR-L1, CDR-L2 et CDR-L3 des séquences SEQ ID No 9, 5 et 11, respectivement, et

ii) les FR1, FR2 et FR3 dérivés de la lignée germinale humaine IGKV1-39*01 (SEQ ID No 47), et

iii) le FR4 dérivé de la lignée germinale humaine IGKJ4*01 (SEQ ID No 49).

Dans un mode de réalisation préféré de l'invention, mais non limitatif, l'anticorps comprend :

a) une chaîne lourde ayant CDR-H1, CDR-H2 et CDR-H3 de séquences SEQ ID No 7, 2 et 3, respectivement, et FR1, FR2 et FR3 dérivées de la lignée germinale humaine IGHV1-46*01 (SEQ ID No 46), et le FR4 dérivé de la lignée germinale humaine IGHJ4*01 (SEQ ID No 48); et

b) une chaîne légère ayant CDR-L1, CDR-L2 et CDR-L3 de séquences SEQ ID No 9, 5 et 11, respectivement, et FR1, FR2 et FR3 dérivées de la lignée germinale humaine IGKV1-39*01 (SEQ ID No 47) et le FR4 dérivé de la lignée germinale humaine IGKJ4*01 (SEQ ID No 49).

Dans un mode de réalisation, l'anticorps comprend un domaine variable de chaîne lourde (VH) de séquence SEQ ID No 33 et un domaine variable de chaîne légère (VL) de séquence SEQ ID No 35. Ledit anticorps humanisé sera appelé ci-après hz208F2 (« Variant 1 » ou « Var. 1 »).

Dans un autre mode de réalisation, l'anticorps comprend un domaine variable de chaîne lourde (VH) de séquence SEQ ID No 33, ladite séquence SEQ ID No 33 comprenant au moins 1 back-mutation choisie parmi les résidus 20, 34, 35, 38, 48, 50, 59, 61, 62, 70, 72, 74, 76, 77, 79, 82 et 95.

Dans un autre mode de réalisation, l'anticorps comprend un domaine variable de chaîne lourde (VH) de séquence SEQ ID No 33, ladite séquence SEQ ID No 33 comprenant 2, 3, 4, 5, 6, 7, 8, 9, 10, 11, 12, 13, 14, 15, 16 ou 17 back-mutations choisies parmi les résidus 20, 34, 35, 38, 48, 50, 59, 61, 62, 70, 72, 74, 76, 77, 79, 82 et 95.

Pour plus de clarté, le tableau 3 suivant illustre les back-mutations préférées.

Tableau 3

| No résidu | 20 | 34 | 35 | 38 | 48 | 50 | 59 | 61 |
|---|---|---|---|---|---|---|---|---|
| Murin | M | I | Y | K | L | W | K | N |
| Humain | V | M | H | R | M | I | S | A |

| No résidu | 62 | 70 | 72 | 74 | 76 | 77 | 79 | 82 | 95 |
|---|---|---|---|---|---|---|---|---|---|
| Murin | E | L | A | K | S | N | A | F | F |
| Humain | Q | M | R | T | T | S | V | E | Y |

Dans un mode de réalisation, l'anticorps comprend un domaine variable de chaîne légère (VL) de séquence SEQ ID No 35, ladite séquence SEQ ID No 35 comprenant au moins 1 back-mutation choisie parmi les résidus 22, 53, 55, 65. 71, 72, 77 et 87.

Dans un mode de réalisation, l'anticorps comprend un domaine variable de chaîne légère (VL) de séquence

SEQ ID No 35, ladite séquence de SEQ ID No 35 comprenant 2, 3, 4, 5, 6, 7 ou 8 back-mutations choisies parmi les résidus 22, 53, 55, 65, 71, 72, 77 ou 87.

Dans un autre mode de réalisation, l'anticorps comprend :

a) un domaine variable (VH) de chaîne lourde de séquence SEQ ID No 33, dans lequel ladite séquence SEQ ID No 33 comprend au moins 1 back-mutation choisie parmi les résidus 20, 34, 35, 38, 48, 50, 59. 61, 62, 70, 72, 74, 76, 77, 79, 82 et 95 ; et

b) un domaine variable (VL) de chaîne légère de séquence SEQ ID No 35, ladite séquence de SEQ ID No 35 comprenant au moins 1 back-mutation choisie parmi les résidus 22, 53, 55, 65, 71, 72, 77 et 87.

Pour plus de clarté, le tableau 4 suivant illustre les back-mutations préférées.

Tableau 4

| No résidu | 22 | 53 | 55 | 65 | 71 | 72 | 77 | 87 |
|---|---|---|---|---|---|---|---|---|
| Murin | S | R | H | R | Y | S | N | F |
| Humain | T | S | Q | S | F | T | S | Y |

Dans un tel mode de réalisation, l'anticorps comprend toutes les back-mutations mentionnées ci-dessus et correspond à un anticorps comprenant un domaine variable de chaîne lourde (VH) de séquence SEQ ID No 34 et un domaine variable de chaîne légère (VL) de séquence SEQ ID No 36. Ledit anticorps humanisé sera appelé ci-après hz208F2 (« Variante 3 » ou « Var. 3 »).

Dans un autre mode de réalisation, toutes les formes humanisées comprises entre la variante 1 et la variante 3 sont également englobées par la présente invention. En d'autres termes, l'anticorps correspond à un anticorps comprenant un domaine variable (VH) de chaîne lourde de séquence « consensus » SEQ ID No 41 et un domaine variable de chaîne légère (VL) de séquence « consensus » SEQ ID No 42. L'anticorps humanisé, dans son ensemble, sera appelé ci-après hz208F2 (« Variant 2 » ou « Var. 2 »).

Dans un mode de réalisation préféré mais non limitatif, l'anticorps est choisi parmi :

a) un anticorps comprenant un domaine variable de chaîne lourde de séquence SEQ ID No 33 ou toute séquence présentant au moins 80%, de préférence 85%, 90%, 95% et 98% d'identité avec la SEQ ID No 33 et les trois chaînes légères CDR de séquences SEQ ID No 9, 5 et 11; et

b) un anticorps comprenant un domaine variable de chaîne lourde de séquence SEQ ID No 34 ou toute séquence présentant au moins 80%, de préférence 85%, 90%, 95% et 98% d'identité avec la SEQ ID No 34 et les trois chaînes légères CDR de séquences SEQ ID No 9, 5 et 11.

Par « toute séquence présentant au moins 80%, de préférence 85%, 90%, 95% et 98% d'identité avec SEQ ID No 33 ou 34 », on entend respectivement les séquences présentant les trois CDR à chaîne lourde SEQ ID No 1, 2 et 3 et, en outre, présentant au moins 80%, de préférence 85%, 90%, 95% et 98%, d'identité avec la séquence complète SEQ ID No 33 ou 34 en dehors des séquences correspondant aux CDR (c'est-à-dire SEQ ID No 1, 2 et 3).

Dans un mode de réalisation de l'invention, l'anticorps est choisi parmi :

a) un anticorps comprenant un domaine variable de chaîne lourde de séquence SEQ ID No 33 ou toute séquence présentant au moins 80% d'identité avec la SEQ ID No 33 et les trois CDR de chaîne légère des séquences SEQ ID No 9, 5 et 11 ; et

b) un anticorps comprenant un domaine variable de chaîne lourde de séquence SEQ ID No 34 ou toute séquence présentant au moins 80% d'identité avec la SEQ ID No 34 et les trois CDR de chaîne légère des séquences SEQ No NO 9, 5 et 11.

S'il n'est pas indiqué dans les paragraphes concernés, dans la présente description, par une séquence quelconque ou par une séquence présentant au moins 80% d'une séquence particulière, il faut comprendre que ladite séquence présente au moins 80% et de préférence 85%, 90%, 95% et 98% d'identité avec la séquence de référence. Que ces séquences contiennent ou non des séquences de CDR, il est entendu que les séquences présentant au moins ces CDR doivent être identiques aux CDR de la séquence de référence, les identités à 80%, de préférence 85%, 90%, 95% et 98%, avec la séquence complète devant être calculées pour la séquence restante située en dehors des séquences

correspondant à ces CDR.

Dans un mode de réalisation préféré, l'anticorps est choisi parmi :

a) un anticorps comprenant un domaine variable de chaîne légère de séquence SEQ ID No 35 ou toute séquence présentant au moins 80%, de préférence 85%, 90%, 95% et 98% d'identité avec la SEQ ID No 35 et les trois chaînes lourdes CDR de séquences SEQ ID No 7, 2 et 3; et

b) un anticorps comprenant un domaine variable de chaîne légère de séquence SEQ ID No 36 ou toute séquence présentant au moins 80%, de préférence 85%, 90%, 95% et 98% d'identité avec la SEQ ID No 36 et les trois chaînes lourdes CDR de séquences SEQ ID No 7, 2 et 3.

Par « toute séquence présentant au moins 80%, de préférence 85%, 90%, 95% et 98% d'identité avec la SEQ ID No 35 ou 36 », on entend désigner les séquences présentant les trois CDR à chaîne légère SEQ ID No 4, 5 et 6 et, en outre, présentant au moins 80%, de préférence 85%, 90%, 95% et 98%, d'identité avec la séquence complète SEQ ID No 35 ou 36 en dehors des séquences correspondant aux CDR (à savoir SEQ ID No 4, 5 et 6).

Dans un mode de réalisation de l'invention, l'anticorps est choisi parmi :

a) un anticorps comprenant un domaine variable de chaîne légère de séquence SEQ ID No 35 ou toute séquence présentant au moins 80% d'identité avec la SEQ ID No 35 et les trois CDR de chaîne lourde de séquences SEQ ID No 7, 2 et 3; et

b) un anticorps comprenant un domaine variable de chaîne lourde de séquence SEQ ID No 36 ou toute séquence présentant au moins 80% d'identité avec la SEQ ID No 36 et les trois CDR de chaîne lourde de séquences SEQ ID No 7, 2 et 3.

Les anticorps humanisés décrits ici peuvent également être caractérisés par le domaine constant et, plus particulièrement, lesdits anticorps humanisés peuvent être sélectionnés notamment parmi IgG1, IgG2, IgG3, IgM, IgA, IgD ou IgE. Plus préférablement, dans le contexte de la présente invention, lesdits anticorps humanisés sont IgG1 ou IgG4.

Selon un mode de réalisation de l'invention, l'anticorps est un anticorps humanisé comprenant les domaines variables VH et VL tels que décrits ci-dessus au format IgG1. Plus préférentiellement, ledit anticorps humanisé comprend un domaine constant pour le VH de séquence SEQ ID No 43 et un domaine Kappa pour le VL de séquence SEQ ID No 45.

Selon un mode de réalisation de l'invention, l'anticorps est un anticorps humanisé comprenant les domaines variables VH et VL tels que décrits ci-dessus au format IgG4. Plus préférentiellement, ledit anticorps humanisé comprend un domaine constant pour le VH de séquence SEQ ID No 44 et un domaine Kappa pour le VL de séquence SEQ ID No 45.

Selon encore un autre mode de réalisation de l'invention, l'anticorps est choisi parmi

a) un anticorps comprenant ou consistant en une chaîne lourde de séquence SEQ ID No 37 ou toute séquence présentant au moins 80% d'identité avec la SEQ ID No 37 et une chaîne légère de séquence SEQ ID No 39 ou toute séquence présentant au moins 80% d'identité avec SEQ ID No 39 ; et

b) un anticorps comprenant ou consistant en une chaîne lourde de séquence SEQ ID No 38 ou toute séquence présentant au moins 80% d'identité avec la SEQ No 38 et une chaîne légère de séquence SEQ ID No 40 ou toute séquence présentant au moins 80% d'identité avec SEQ ID No 40.

Pour plus de clarté, le tableau 5a suivant illustre des exemples non limitatifs de séquences de VH et VL pour le variant 1 (Var. 1) et le variant 3 (Var. 3) de l'anticorps humanisé hz208F2. Il comprend également la séquence consensus pour le variant 2 (Var. 2).

Tableau 5a

| | Chaine lourde | Chaine légère | SEQ ID No. |
|---|---|---|---|
| hz208F2 (var. 1) | Domaine variable (VH) | | 33 |
| | | Domaine variable (VL) | 35 |
| | Longueur complète | | 37 |
| | | Longueur complète | 39 |

(suite)

| | Chaine lourde | Chaine légère | SEQ ID No. |
|---|---|---|---|
| hz208F2 (Var. 3) | Domaine variable (VH) | | 34 |
| | | Domaine variable (VL) | 36 |
| | Longueur complète | | 38 |
| | | Longueur complète | 40 |
| hz208F2 (Var. 2) | Domaine variable (VH) | | 41 |
| | | Domaine variable (VL) | 42 |

Dans un autre mode de réalisation préféré, l'anticorps est choisi parmi :

a) un anticorps comprenant un domaine variable de séquence de chaîne lourde choisi parmi les SEQ ID No 56, 62, 64, 66, 68, 70, 72, 74, 76, 78 et 54 ou toute séquence d'au moins 80%, de préférence d'au moins 85%, 90%, 95% et 98% d'identité avec SEQ ID No 56, 62, 64, 66, 68, 70, 72, 74, 76, 78 et 54; et les trois CDR de chaînes légères de séquences SEQ ID No 9, 5 et 11;

b) un anticorps comprenant un domaine variable de séquence de chaîne légère choisi parmi les SEQ ID No 57 ou 60 ou toute séquence présentant au moins 80%, de préférence au moins 85%, 90%, 95% et 98% d'identité avec SEQ ID No 57 ou 60; et les trois CDR de chaînes lourdes de séquences SEQ ID No 7, 2 et 3; et

c) un anticorps comprenant un domaine variable de séquence de chaîne lourde choisi parmi les SEQ ID No 56, 62, 64, 66, 68, 70, 72, 74, 76, 78 et 54 ou toute séquence d'au moins 80%, de préférence d'au moins 85 %, 90%, 95% et 98% d'identité avec les SEQ ID No 56, 62, 64, 66, 68, 70, 72, 74, 76, 78 et 54; et un domaine variable de chaîne légère choisi parmi SEQ ID No 57 ou 60 ou toute séquence ayant au moins 80%, de préférence au moins 85%, 90%, 95% et 98% d'identité avec SEQ ID No 57 ou 60.

Selon encore un autre mode de réalisation de l'invention, l'anticorps est choisi parmi:

a) un anticorps comprenant une chaîne lourde de séquence SEQ ID No 56, 62, 64, 66, 68, 70, 72, 74, 76, 78 et 54 ou toute séquence présentant au moins 80% d'identité avec la SEQ ID No 56, 62, 64, 66, 68, 70, 72, 74, 76, 78 ou 54, et une chaîne légère de séquence SEQ ID No 57 ou toute séquence présentant au moins 80% d'identité avec la SEQ ID No 57 ; et
b) un anticorps comprenant une chaîne lourde de séquence SEQ ID No 56, 64, 68 et 78 ou toute séquence présentant au moins 80% d'identité avec la SEQ ID No 56, 64, 68 ou 78 et une chaîne légère de séquence SEQ ID No 60, ou toute séquence présentant au moins 80% d'identité avec la SEQ ID No 60.

Selon encore un autre mode de réalisation de l'invention, l'anticorps est choisi parmi :

a) un anticorps comprenant ou consistant en une chaîne lourde de séquence SEQ ID No 58 ou toute séquence présentant au moins 80% d'identité avec la SEQ ID No 58 et une chaîne légère de séquence SEQ ID No 59 ou toute séquence présentant au moins 80% d'identité avec SEQ ID No 59 ;

b) un anticorps comprenant ou consistant en une chaîne lourde de séquence SEQ ID No 58 ou toute séquence présentant au moins 80% d'identité avec la SEQ ID No 58 et une chaîne légère de séquence SEQ ID No 61 ou toute séquence présentant au moins 80% d'identité avec SEQ ID No 61 ;

c) un anticorps comprenant ou consistant en une chaîne lourde de séquence SEQ ID No 63 ou toute séquence présentant au moins 80% d'identité avec la SEQ ID No 63 et une chaîne légère de séquence SEQ ID No 59 ou toute séquence présentant au moins 80% d'identité avec SEQ ID No 59 ;

d) un anticorps comprenant ou consistant en une chaîne lourde de séquence SEQ ID No 65 ou toute séquence présentant au moins 80% d'identité avec la SEQ ID No 65 et une chaîne légère de séquence SEQ ID No 59 ou toute séquence présentant au moins 80% % d'identité avec SEQ ID No 59 ;

e) un anticorps comprenant ou consistant en une chaîne lourde de séquence SEQ ID No 65 ou toute séquence présentant au moins 80% d'identité avec la SEQ ID No 65 et une chaîne légère de séquence SEQ ID No 61 ou toute séquence présentant au moins 80% d'identité avec SEQ ID No 61 ;

f) un anticorps comprenant ou consistant en une chaîne lourde de séquence SEQ ID No 67 ou toute séquence présentant au moins 80% d'identité avec la SEQ ID No 67 et une chaîne légère de séquence SEQ ID No 59 ou toute séquence présentant au moins 80% d'identité avec SEQ ID No 59 ;

g) un anticorps comprenant ou consistant en une chaîne lourde de séquence SEQ ID No 69 ou toute séquence présentant au moins 80% d'identité avec la SEQ ID No 69 et une chaîne légère de séquence SEQ ID No 59 ou toute séquence présentant au moins 80% d'identité avec SEQ ID No 59 ;

h) un anticorps comprenant ou consistant en une chaîne lourde de séquence SEQ ID No 69 ou toute séquence présentant au moins 80% d'identité avec la SEQ ID No 69 et une chaîne légère de séquence SEQ ID No 61 ou toute séquence présentant au moins 80% d'identité avec SEQ ID No 61 ;

i) un anticorps comprenant ou consistant en une chaîne lourde de séquence SEQ ID No 71 ou toute séquence présentant au moins 80% d'identité avec la SEQ ID No 71 et une chaîne légère de séquence SEQ ID No 59 ou toute séquence présentant au moins 80% d'identité avec SEQ ID No 59 ;

j) un anticorps comprenant ou consistant en une chaîne lourde de séquence SEQ ID No 73 ou toute séquence présentant au moins 80% d'identité avec la SEQ ID No 73 et une chaîne légère de séquence SEQ ID No 59 ou toute séquence présentant au moins 80% d'identité avec SEQ ID No 59 ;

k) un anticorps comprenant ou consistant en une chaîne lourde de séquence SEQ ID No 75 ou toute séquence présentant au moins 80% d'identité avec la SEQ ID No 75 et une chaîne légère de séquence SEQ ID No 59 ou toute séquence présentant au moins 80% d'identité avec SEQ ID No 59 ;

l) un anticorps comprenant ou consistant en une chaîne lourde de séquence SEQ ID No 77 ou toute séquence présentant au moins 80% d'identité avec la SEQ ID No 77 et une chaîne légère de séquence SEQ ID No 59 ou toute séquence présentant au moins 80% d'identité avec SEQ ID No 59 ;

m) un anticorps comprenant ou consistant en une chaîne lourde de séquence SEQ ID No 53 ou toute séquence présentant au moins 80% d'identité avec la SEQ ID No 53 et une chaîne légère de séquence SEQ ID No 59 ou toute séquence présentant au moins 80% d'identité avec SEQ ID No 59 ;

n) un anticorps comprenant ou consistant en une chaîne lourde de séquence SEQ ID No 53 ou toute séquence présentant au moins 80% d'identité avec la SEQ ID No 53 et une chaîne légère de séquence SEQ ID No 61 ou toute séquence présentant au moins 80% d'identité avec SEQ ID No 61 ; et

o) un anticorps comprenant ou consistant en une chaîne lourde de séquence SEQ ID No 55 ou toute séquence présentant au moins 80% d'identité avec la SEQ ID No 55 et une chaîne légère de séquence SEQ ID No 59 ou toute séquence présentant au moins 80% d'identité avec SEQ ID No 59.

En d'autres termes, l'anticorps peut être un anticorps comprenant :

a) une chaîne lourde de séquence sélectionnée parmi les SEQ ID No 58, 63, 65, 67, 69, 71, 73, 75, 77, 53 et 55 ou toute séquence présentant au moins 80% d'identité avec la SEQ ID No 58, 63, 65, 67, 69, 71, 73, 75, 77, 53 et 55 ; et

b) une chaîne légère de séquence sélectionnée parmi les SEQ ID No 59 et 61 ou toute séquence présentant au moins 80% d'identité avec les SEQ ID No 59 et 61.

Dans un mode de réalisation de l'invention, l'anticorps est choisi parmi :

a) une chaîne lourde de séquence sélectionnée parmi les SEQ ID No 58, 63, 65, 67, 69, 71, 73, 75, 77, 53 et 55 ou toute séquence présentant au moins 80% d'identité avec la SEQ ID No 58, 63, 65, 67, 69, 71, 73, 75, 77, 53 ou 55; et

b) une chaîne légère de séquence sélectionnée parmi les SEQ ID No 59 et 61 ou toute séquence présentant au

moins 80% d'identité avec la SEQ ID No 59 ou 61.

Pour plus de clarté, le tableau 5b suivant illustre des exemples non limitatifs de séquences de VH et VL (domaine variable et longueur complète) pour différents variants de l'anticorps humanisé hz208F2.

Table 5b

| | Chaine lourde | Chaine légère | SEQ ID NO. |
|---|---|---|---|
| hz208F2 H037/L018 | Domaine variable (VH) | | 56 |
| | | Domaine variable (VL) | 57 |
| | Longueur complète | | 58 |
| | | Longueur complète | 59 |
| Hz208F2 H037/L021 | Domaine variable (VH) | | 56 |
| | | Domaine variable (VL) | 60 |
| | Longueur complète | | 58 |
| | | Longueur complète | 61 |
| Hz208F2 H047/L018 | Domaine variable (VH) | | 62 |
| | | Domaine variable (VL) | 57 |
| | Longueur complète | | 63 |
| | | Longueur complète | 59 |
| Hz208F2 H049/L018 | Domaine variable (VH) | | 64 |
| | | Domaine variable (VL) | 57 |
| | Longueur complète | | 65 |
| | | Longueur complète | 59 |
| Hz208F2 H049/L021 | Domaine variable (VH) | | 64 |
| | | Domaine variable (VL) | 60 |
| | Longueur complète | | 65 |
| | | Longueur complète | 61 |
| Hz208F2 H051/L018 | Domaine variable (VH) | | 66 |
| | | Domaine variable (VL) | 57 |
| | Longueur complète | | 67 |
| | | Longueur complète | 59 |
| Hz208F2 H052/L018 | Domaine variable (VH) | | 68 |
| | | Domaine variable (VL) | 57 |
| | Longueur complète | | 69 |
| | | Longueur complète | 59 |
| Hz208F2 H052/L021 | Domaine variable (VH) | | 68 |
| | | Domaine variable (VL) | 60 |
| | Longueur complète | | 69 |
| | | Longueur complète | 61 |
| Hz208F2 H057/L018 | Domaine variable (VH) | | 70 |
| | | Domaine variable (VL) | 57 |
| | Longueur complète | | 71 |
| | | Longueur complète | 59 |
| Hz208F2 H068/L018 | Domaine variable (VH) | | 72 |
| | | Domaine variable (VL) | 57 |
| | Longueur complète | | 73 |
| | | Longueur complète | 59 |
| Hz208F2 | Domaine variable (VH) | | 74 |
| | | Domaine variable (VL) | 57 |

(suite)

| | Chaine lourde | Chaine légère | SEQ ID NO. |
|---|---|---|---|
| H070/L018 | Longueur complète | | 75 |
| | | Longueur complète | 59 |
| | Domaine variable (VH) | | 76 |
| Hz208F2 | | Domaine variable (VL) | 57 |
| H071/L018 | Longueur complète | | 77 |
| | | Longueur complète | 59 |
| | Domaine variable (VH) | | 78 |
| Hz208F2 | | Domaine variable (VL) | 57 |
| H076/L018 | Longueur complète | | 53 |
| | | Longueur complète | 59 |
| | Domaine variable (VH) | | 78 |
| Hz208F2 | | Domaine variable (VL) | 60 |
| H076/L021 | Longueur complète | | 53 |
| | | Longueur complète | 61 |
| | Domaine variable (VH) | | 54 |
| Hz208F2 | | Domaine variable (VL) | 57 |
| H077/L018 | Longueur complète | | 55 |
| | | Longueur complète | 59 |

Selon un autre aspect de la présente invention, l'anticorps est un anticorps produit par l'hybridome I-4757, I-4773, I-4775, I-4736 ou I-4774 déposé à la CNCM de l'Institut Pasteur France les 30 mai 2013, 26 juin 2013, 26 juin 2013, 24 avril 2013 et 26 juin 2013, respectivement.

L'anticorps est plus particulièrement un anticorps 208F2 (par ex. chimérique ou humanisé, notamment chimérique) et plus particulière un anticorps c208F2 qui est un anticorps monoclonal (IgG1). Cet anticorps est notamment décrit dans WO2015162291 et est caractérisé en ce qu'il comprend les trois CDR de chaine légère de séquence SEQ ID Nos. 9, 5 et11 respectivement, et les trois CDR de chaine lourde de séquence SEQ ID Nos 7, 2 et 3 respectivement. L'anticorps est en outre caractérisé en ce qu'il comprend un domaine variable de la chaine légère de séquence SEQ ID No 18 et un domaine variable de chaine lourde de séquence SEQ ID No 13. L'anticorps est enfin caractérisé en ce qu'il comprend une chaine légère de séquence SEQ ID No 28 et un domaine variable de chaine lourde de séquence SEQ ID No 23.

L'anticorps c208F2 et l'ADC-A peuvent être obtenus par toutes les méthodes connues de l'homme du métier d'obtention d'anticorps et d'ADC. Ils sont notamment obtenus par les méthodes décrites dans la demande WO2015162291 (ADC-A correspond de préférence à l'ADC nommé c208F2-G-13 dans WO2015162291).

Traitement

Par « résistance » selon l'invention, on entend que le patient ne présente plus de réponse au traitement, c'est-à-dire qu'il y a reprise de la progression du cancer ou stagnation.

Par « traitement » selon l'invention, on entend notamment une augmentation de la survie globale, et/ou une augmentation de la durée de survie sans progression et/ou une augmentation de la survie sans aggravation et/ou une diminution de la récurrence et/ou une diminution de la taille de la tumeur.

L'évaluation de ces critères de réponse tumorale (résistance et traitement) est bien connue de l'homme du métier et pourra notamment être mesurée par les critères RECIST (Eisenhauer et al Eur J Cancer, 45 (2009) : 228-247 ou ses mises à jour).

La maladie proliférative sera plus particulièrement un cancer, et notamment un cancer résistant au docétaxel.

Le cancer selon l'invention pourra notamment être sélectionné parmi le cancer du sein, du colon, mélanome, du poumon dont du poumon non à petites cellules, gastrique, des voies aéro-digestives supérieures, de l'oesophage, colorectal, gastrique, gliome, de l'oesophage, de l'ovaire, de la prostate, rénal, de la thyroïde, de l'utérus, oral squameux, mésothéliome.

Dans un mode de réalisation particulier, le cancer traité selon l'invention sera un cancer habituellement traité par le docétaxel notamment les cancers gastriques, de la prostate, du poumon (dont cancer du poumon non à petites

cellules), du sein et des voies aéro-digestives supérieures.

Dans un mode de réalisation particulier, le cancer traité selon l'invention sera un cancer comprenant des cellules tumorales exprimant ou sur-exprimant tout ou partie de la protéine IGF-1R. Les cancers exprimant IGF-1R pourront être des cancers exprimant initialement IGF-1R ou des cancers qui n'exprimait pas initialement IGF-1R mais qui se sont mis à l'exprimer après être devenu résistant à un premier traitement (ou plus), tel que résistant au docétaxel.

Dans un mode de réalisation, l'ADC-A pourra être administré après apparition d'une résistance au docétaxel. ADC-A pourra alors être administré seul, postérieurement au docétaxel ou de façon concomitante avec le docétaxel et/ou une autre chimiothérapie. L'initiation du traitement par ADC-A pourra notamment débuter immédiatement après la constatation de la survenue de la résistance, ou dans les 1, 2, 3, 4, 5, 6, 7, ou encore 8 semaines ou plus après la constatation de la survenue de la résistance, notamment avant 16 semaines suivant la constatation de la survenue de la résistance.

Dans un autre mode de réalisation, l'ADC-A pourra être administré avant l'apparition de la résistance au docétaxel, de manière concomitante au docétaxel. L'initiation du traitement par ADC-A pourra alors être initié en même temps ou après le traitement au docétaxel mais avant l'apparition d'une résistance au docétaxel..

Par « de manière concomitante » on entend que les produits sont administrés de manière à être présent en même temps dans l'organisme du patient. Les produits peuvent être administrés en même temps ou de manière séquentielle.

Par « de manière postérieure » ou « postérieurement » on entend que l'initiation du traitement par le premier produit suit le traitement par le second produit, par exemple que le traitement par ADC-A suit le traitement par le docétaxel. Les deux produits ne sont donc pas simultanément présents dans l'organisme du patient.

La posologie du docétaxel dépendra de la gravité, de l'état du patient et du type de cancer à traiter. Elle pourra aussi varier en fonction des traitements préalablement ou concomitamment reçus par le patient. De manière générale, le docétaxel sera administré à une dose comprise entre 50mg/$m^2$ de surface corporelle (BSA - Body surface area) et 125 mg/$m^2$ notamment entre 75 mg/$m^2$ et 100 mg/$m^2$ toute les 1 à 4 semaines notamment toutes les trois semaines.

Le nombre de cycles de traitement (c'est-à-dire le nombre d'occurrences de l'administration du traitement et du temps entre deux administrations) dépendra de la réponse du patient et de l'apparition d'une résistance au docétaxel. Il pourra notamment être compris entre 3 et 12 cycles ou plus.

La posologie de l'ADC-A dépendra de la gravité, de l'état du patient et du type de cancer à traité. Elle pourra aussi varier en fonction des traitements antérieurement ou concomitamment reçus par le patient. De manière générale, ADC-A sera administré à une dose comprise entre 0,5 et 3 mg/kg de patient à traiter, notamment entre 0,9 et 2,5 mg/kg de patient à traiter, toutes les 1 à 4 semaines notamment toutes les trois semaines.

Le nombre de cycles de traitement par ADC-A dépendra notamment de la réponse du patient. Il pourra notamment être compris entre 1 et 18 cycles ou plus jusqu'à reprise de la progression de la tumeur ou jusqu'à la rémission

Le traitement selon l'invention pourra être combiné avec d'autres traitements anti-cancéreux préalablement et/ou concomitamment au docétaxel et/ ou à l'ADC-A. Notamment le traitement anti-cancéreux est un traitement avec une molécule choisie dans le groupe constitué par la doxorubicine, le cyclophosphamide, la capécitabine, le cisplatine, le paclitaxel, la carboplatine, le lapatinib, le pertuzimab, le bevacizumab, le trastuzumab, le 5-fluorouracile, l'anthracycline, la vinorelbine, le binimetinib, l'encorafenib et le neratinib. Le traitement selon l'invention pourra aussi être combiné avec d'autres types de médicaments tels que les corticoïdes, notamment la dexaméthasone ou la prednisone.

La présente invention porte en outre sur une composition pharmaceutique comprenant ADC-A et au moins un excipient pharmaceutiquement acceptable pour son utilisation selon l'invention. Notamment ADC-A sera formulé de manière à pouvoir être injecté par voie intraveineuse.

## EXEMPLE :

### Matériel et méthode

Cellules : les cellules MCF-7 sont obtenues de l'ATTC (Manassas). Les cellules sont maintenues dans un incubateur à 5 % de CO2, 90 % d'humidité et 37°C dans un milieu de culture cellulaire standard, tel que recommandé par le fournisseur.

Génération de l'anticorps : L'anticorps 208F2 est produit comme décrit dans la demande WO2015162291. Succinctement, l'anticorps anti-IGF-1R, c208F2 humanisé (hz208F2(4)) est généré en utilisant la technologie des hybridomes. Des souris Balb/c sont immunisées avec la protéine humaine recombinante IGF-1R (rhIGF-1R) (systèmes R&D) en présence d'adjuvant de Freund. Les rates sont fusionnées avec le partenaire de fusion du myélome SP2/0. Après le clonage par dilution limite, la liaison et l'internalisation de l'anticorps aux cellules MCF-7 est confirmée et l'isotype déterminé. La spécificité de liaison est vérifiée par ELISA sur du rhIGF-1R, du récepteur recombinant de l'insuline humaine (rhIR) et de l'IGF-1R murin (mIGF-1R) (systèmes R&D). L'anticorps m208F2 est ensuite humanisé par greffe des régions déterminant la complémentarité (CDR) et exprimé dans des cellules ovariennes de hamster chinois pour

une caractérisation pharmacologique complète.

Production et caractérisation de ADC-A : ADC-A est produit comme décrit dans la demande WO2015162291 (ADC-A correspond à l'ADC nommé c208F2-G-13 de WO2015162291). Une légère réduction du mAb 208F2 (hz208F2-4) et le couplage au linker payload est effectué comme décrit précédemment (Sun et al. Bioconjug. Chem 2005 ; 16 :1282-90, Wagner-Rousset et al Mabs 2014 ; 6 :173-84). Brièvement, pour cibler un DAR de 4, le hz208F2-4 a été réduit avec 2,5 équivalents molaires de tris(2-carboxyéthyl)phosphine (TCEP). L'anticorps réduit a ensuite été traité avec 7 équivalents molaires de linker-payload (G-13). Après conjugaison, ADC-A est concentré à 5 mg/mL (dans du tampon dans 25 mM d'histidine pH 6,5,150 mM NaCl et 6% de saccharose). Du Tween 80 a ensuite été ajouté pour obtenir une concentration finale de 0,005% (v/v). Le produit est finalement filtré à travers un filtre Stericup (GP Express PLUS Membrane, 0.22μm, Polyethersulfone, Millipore) et conservé à 4°C.

Détermination de l'activité in vivo : L'activité in vivo est testée dans un modèle de cancer du sein : des souris nudes femelles de 7 semaines (Charles River Laboratories, n=15) ont été greffées par voie sous-cutanée avec $5 \times 10^6$ cellules MCF-7 et ce 1 jour après implantation sous-cutanée de 0,72 mg de granules libérant pendant 60 jours du 17β-estradiol (Innovative Research of America).

Les souris sont randomisées, et le traitement est amorcé lorsque les tumeurs atteignent une taille d'environ 150 $mm^3$. Le volume tumoral (longueur × largeur × hauteur × 0,52) est mesuré au moins deux fois par semaine et la réponse thérapeutique est définie à l'aide des critères d'évaluation de la réponse des tumeurs solides (RECIST).

Les souris porteuses de tumeurs MCF-7 sont injectées par voie intraveineuse avec du docétaxel, 9 mg/kg deux fois par semaine, avec un total de 5 injections. Lorsque les tumeurs sont devenues résistantes au docétaxel, les souris sont divisées en 2 groupes de 5 animaux : le groupe 1 reçoit 9 mg/kg de docétaxel toutes les 2 semaines et le groupe 2 reçoit une injection unique de ADC-A de 3 mg/kg.

Le pourcentage des valeurs de régression est calculé à l'aide de la formule suivante :

$$\text{Régression } \% = 100 \times \Delta T / T\text{initial}$$

où T = le volume de la tumeur dans le groupe traité, ΔT = le volume de la tumeur dans le groupe traité le jour de l'étude moins le volume de la tumeur dans le groupe traité au jour initial de l'administration, et Tinitial = le volume de la tumeur dans le groupe traité au jour initial de l'administration. La maladie est considérée comme évolutive lorsque la taille de la tumeur augmente de > 20 %. La régression partielle (RP) est définie comme une diminution de la taille de la tumeur > 30 %. L'absence de croissance tumorale, ou une légère diminution (< 30 %), ou une légère augmentation (< 20 %) de la taille de la tumeur est définie comme une maladie stable (ET), et une absence de masse tumorale palpable est définie comme une régression complète (RC).

## Résultats

Afin d'évaluer le potentiel thérapeutique de ADC-A dans le cancer du sein, l'effet de l'administration d'ADC-A a été étudié dans un modèle de souris greffées par des cellules MCF-7 après apparition d'une résistance au docétaxel. Après 70 jours, les tumeurs sont devenues résistantes au docétaxel et ont rechuté (Fig. 1). L'administration d'une dose unique d'ADC-A de 3 mg/kg chez des souris atteintes de tumeurs résistantes au docétaxel induit une inhibition forte et significative de la croissance tumorale (P 0,05) (Fig. 1), On observe une RC chez 1 souris et une RP chez 3 souris sur 5. ADC-A et le docétaxel présente un effet synergique. On peut en conclure que ADC-A est un bon traitement seul ou en combinaison avec le docétaxel pour le traitement des tumeurs résistantes au docétaxel. Ceci est d'autant plus surprenant que le payload utilisé à un mécanisme d'action proche (action sur la tubuline) des taxanes et donc du docétaxel.

## Revendications

**1.** ADC de formule (I) suivante

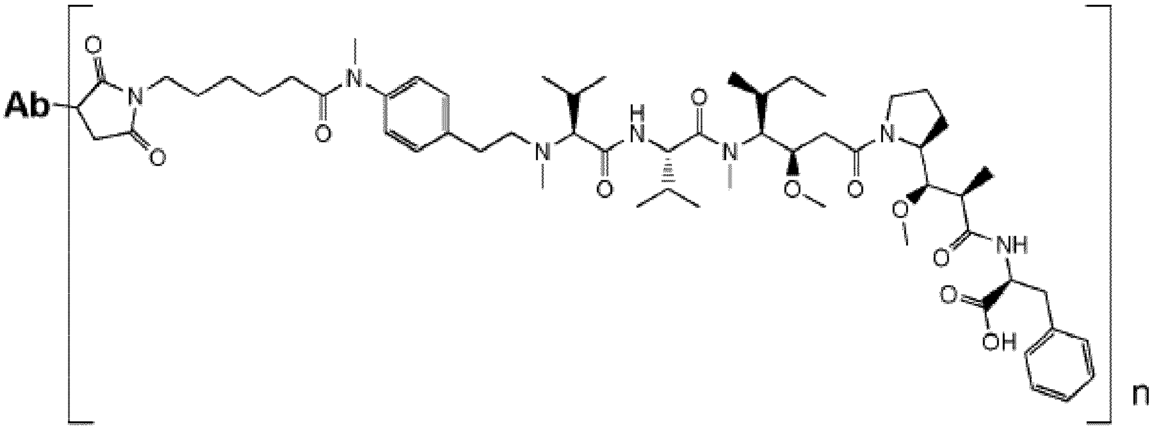

(I)

dans laquelle Ab est un anticorps anti-IGF1R **caractérisé en ce qu'**il comprend des CDR de chaine légère de séquences SEQ ID Nos. 9, 5 et 11 respectivement et des CDR de chaine lourde de séquence SEQ ID Nos 7, 2 et 3 respectivement, et n est compris entre 1 et 12,
pour son utilisation dans le traitement de maladies prolifératives, telle que le cancer, **caractérisée en ce que** l'ADC est administré de façon concomitante ou postérieurement à du docétaxel.

**2.** ADC pour son utilisation selon la revendication 1, **caractérisé en ce que** Ab est un anticorps comprenant un domaine variable de chaine légère de séquence SEQ ID No 18 et un domaine variable de chaine lourde de séquence SEQ ID No 13.

**3.** ADC pour son utilisation selon la revendication 1 ou 2, **caractérisé en ce que** l'ADC est administré à un patient ayant développé une résistance au docétaxel.

**4.** ADC pour son utilisation selon l'une quelconque des revendications 1 à 3, **caractérisé en ce que** le traitement par l'ADC a une durée comprise entre 1 mois et 5 ans.

5. ADC pour son utilisation selon l'une quelconque des revendications 1 à 4, dans laquelle l'ADC est administré immédiatement après la constatation de la survenue de la résistance au docétaxel, ou dans les 1, 2, 3, 4, 5, 6, 7, ou encore 8 semaines ou plus après la constatation de la survenue de la résistance, notamment avant 16 semaines suivant la constatation de la survenue de la résistance.

6. ADC pour son utilisation selon l'une quelconque des revendications 1 à 5, **caractérisé en ce que** l'ADC est administré par cycle à une dose comprise entre 1 et 2 mg/kg de patient à traiter toutes les 2 à 4 semaines

7. ADC pour son utilisation selon l'une quelconque des revendication 1 à 6, **caractérisé en ce que** le nombre de cycles est compris entre 1 et 18 cycles.

8. ADC pour son utilisation selon l'une quelconque des revendications 1 à 7, **caractérisé en ce que** le traitement du cancer est mesuré par une augmentation de la survie globale, et/ou une augmentation de la durée de survie sans progression et/ou une augmentation de la survie sans aggravation et/ou une diminution de la récurrence et/ou une diminution de la taille de la tumeur.

9. ADC pour son utilisation selon l'une quelconque des revendication 1 à 8, **caractérisé en ce que** l'ADC est administré à un patient ayant été traité, préalablement ou concomitamment au docétaxel et/ ou à l'ADC, par une ou plusieurs autres chimiothérapies, notamment à l'aide d'une ou plusieurs molécules choisies dans le groupe constitué par la doxorubicine, le cyclophosphamide, la capécitabine, le cisplatine, le paclitaxel, la carboplatine, le lapatinib, le pertuzimab, le bevacizumab, le trastuzumab, le 5-fluorouracile, l'anthracycline, le binimetinib, l'encorafenib et le neratinib.

10. Composition pharmaceutique comprenant un ADC tel que défini selon l'une quelconque des revendications 1 et 2 et au moins un excipient pharmaceutiquement acceptable pour son utilisation dans le traitement de maladies prolifératives, telle que le cancer, **caractérisée en ce que** la composition pharmaceutique comprenant l'ADC est administrée de façon concomitante ou postérieurement à du docétaxel.

11. Composition pharmaceutique pour son utilisation selon la revendication 10, **caractérisée en ce que** la maladie proliférative est un cancer résistant au docétaxel.

12. Composition pharmaceutique pour son utilisation selon la revendication 10 ou 11, **caractérisée en ce qu'**elle est administrée immédiatement après la constatation de la survenue de la résistance au docétaxel, ou dans les 1, 2, 3, 4, 5, 6, 7, ou encore 8 semaines ou plus après la constatation de la survenue de la résistance, notamment avant 16 semaines suivant la constatation de la survenue de la résistance.

13. Composition pharmaceutique pour son utilisation selon l'une quelconque des revendications 10 à 12, **caractérisée en ce qu'**elle est administrée par cycle à une dose en ADC comprise entre 1 et 2 mg/kg de patient à traiter toutes les 2 à 4 semaines, notamment avec un nombre de cycles compris entre 1 et 18 cycles.

14. Composition pharmaceutique pour son utilisation selon l'une quelconque des revendications 10 à 13, **caractérisée en ce que** le traitement par la composition pharmaceutique a une durée comprise entre 1 mois et 5 ans.

15. Composition pharmaceutique pour son utilisation selon l'une quelconque des revendications 10 à 14, **caractérisée en ce que** la composition pharmaceutique est administré à un patient ayant été traité, préalablement ou concomitamment au docétaxel et/ou à l'ADC, par une ou plusieurs autres chimiothérapies, notamment à l'aide d'une ou plusieurs molécules choisies dans le groupe constitué par la doxorubicine, le cyclophosphamide, la capécitabine, le cisplatine, le paclitaxel, la carboplatine, le lapatinib, le pertuzimab, le bevacizumab, le trastuzumab, le 5-fluorouracile, l'anthracycline, le binimetinib, l'encorafenib et le neratinib.

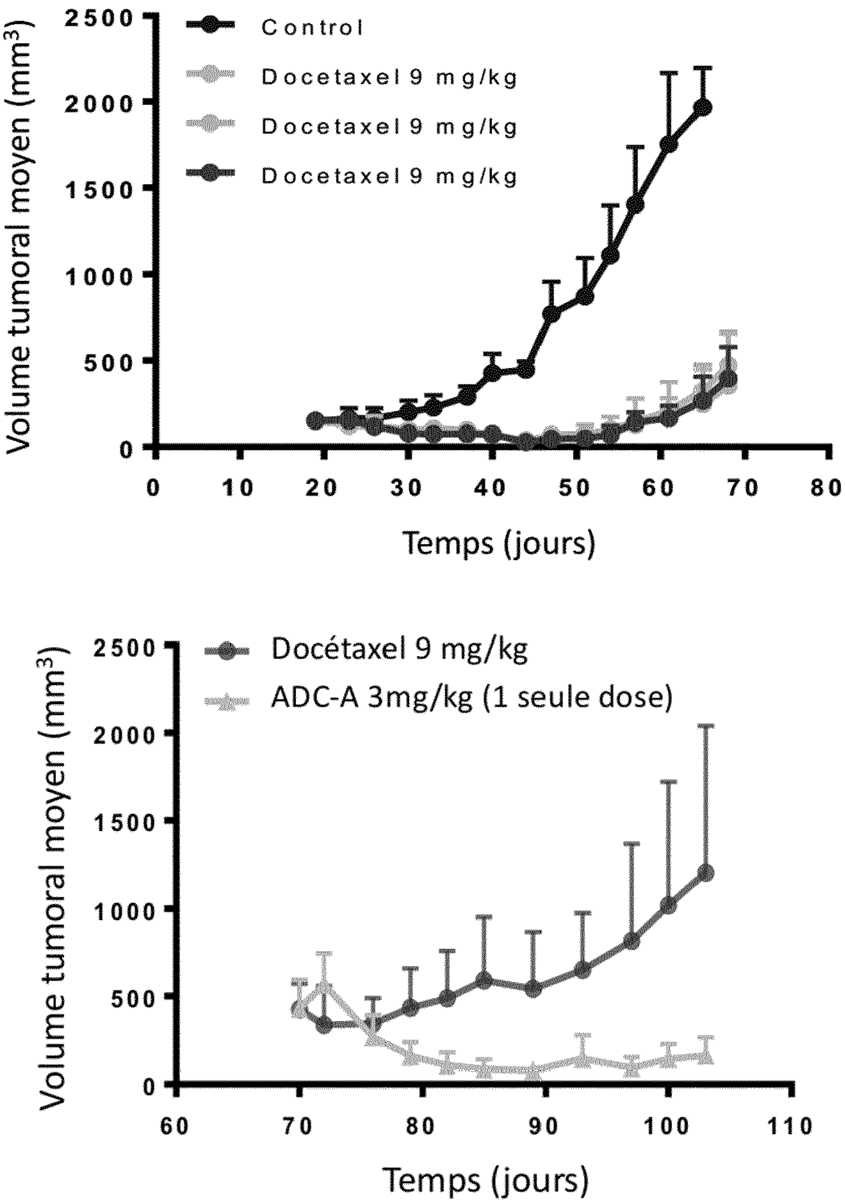
Control
Docetaxel 9 mg/kg
Docetaxel 9 mg/kg
Docetaxel 9 mg/kg
Volume tumoral moyen (mm3)
2500
2000
1500
1000
500
0
0 10 20 30 40 50 60 70 80
Temps (jours)
Docétaxel 9 mg/kg
ADC-A 3mg/kg (1 seule dose)
Volume tumoral moyen (mm3)
2500
2000
1500
1000
500
0
60 70 80 90 100 110
Temps (jours)

A.

B.

FIG. 1

Europäisches Patentamt
European Patent Office
Office européen des brevets

# RAPPORT DE RECHERCHE EUROPEENNE

Numéro de la demande
EP 19 30 5578

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | WO 2015/162291 A1 (PF MEDICAMENT [FR]) 29 octobre 2015 (2015-10-29)<br>* page 181; composé G13 *<br>* pages 189,190 *<br>----- | 1-15 | INV.<br>A61K47/68<br>A61P35/00 |
| A | HOANG BRYAN ET AL: "Cabazitaxel-conjugated nanoparticles for docetaxel-resistant and bone metastatic prostate cancer", CANCER LETTERS, vol. 410, 2017, pages 169-179, XP085271190, ISSN: 0304-3835, DOI: 10.1016/J.CANLET.2017.09.029<br>* page 171; figure 1 *<br>----- | 1-15 | |

DOMAINES TECHNIQUES RECHERCHES (IPC)

A61K

Le présent rapport a été établi pour toutes les revendications

1

| Lieu de la recherche | Date d'achèvement de la recherche | Examinateur |
|---|---|---|
| Munich | 8 novembre 2019 | Trifilieff-Riolo, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 19 30 5578

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-11-2019

| Document brevet cité au rapport de recherche | Date de publication | Membre(s) de la famille de brevet(s) | Date de publication |
|---|---|---|---|
| WO 2015162291 A1 | 29-10-2015 | AU 2015250759 A1 | 10-11-2016 |
| | | BR 112016024619 A2 | 10-10-2017 |
| | | CA 2946469 A1 | 29-10-2015 |
| | | CN 106456800 A | 22-02-2017 |
| | | DK 3134124 T3 | 06-05-2019 |
| | | EP 3134124 A1 | 01-03-2017 |
| | | EP 3498301 A1 | 19-06-2019 |
| | | ES 2727103 T3 | 14-10-2019 |
| | | HR P20190888 T1 | 12-07-2019 |
| | | JP 6258523 B2 | 10-01-2018 |
| | | JP 2017513900 A | 01-06-2017 |
| | | KR 20160146817 A | 21-12-2016 |
| | | LT 3134124 T | 27-05-2019 |
| | | MA 39909 B1 | 31-05-2019 |
| | | PL 3134124 T3 | 30-09-2019 |
| | | SI 3134124 T1 | 28-06-2019 |
| | | TN 2016000472 A1 | 04-04-2018 |
| | | US 2017043031 A1 | 16-02-2017 |
| | | WO 2015162291 A1 | 29-10-2015 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2015162291 A


### Littérature non-brevet citée dans la description


- **EISENHAUER et al.** *Eur J Cancer,* 2009, vol. 45, 228-247
- **SUN et al.** *Bioconjug. Chem,* 2005, vol. 16, 1282-90
- **WAGNER-ROUSSET et al.** *Mabs,* 2014, vol. 6, 173-84